# EUROPEAN PATENT SPECIFICATION

(11) **EP 3 218 517 B1**
(45) Date of publication and mention of the grant of the patent: **01.05.2024**
(21) Application number: 15858318.7
(22) Date of filing: 10.11.2015
(51) Int. Cl.: A61K 48/00, G01N 33/50, A61K 31/7088, A61P 1/00, A61P 1/04, A61P 35/00, A61K 31/7105

(54) **MIR-214 AS A DIAGNOSTIC AND PROGNOSTIC BIOMARKER SPECIFIC FOR ULCERATIVE COLITIS AND A MIR-214 INHIBITOR FOR TREATMENT OF ULCERATIVE COLITIS AND COLITIS-ASSOCIATED COLORECTAL CANCER**
MIR-214 ALS DIAGNOSTISCHER UND PROGNOSTISCHER SPEZIFISCHER BIOMARKER FÜR COLITIS ULCEROSA UND MIR-214-HEMMER ZUR BEHANDLUNG VON COLITIS ULCEROSA UND KOLITIS-ASSOZIIERTER KOLOREKTALER KREBS
MIR-214 COMME BIOMARQUEUR DE DIAGNOSTIC ET DE PRONOSTIC SPÉCIFIQUE À LA RECTOCOLITE HÉMORRAGIQUE ET INHIBITEUR DE MIR-214 POUR SON UTILISATION DANS LE TRAITEMENT DE LA RECTOCOLITE HÉMORRAGIQUE ET DU CANCER COLORECTAL ASSOCIÉ À LA COLITE

(30) Priority: 10.11.2014 US 201462077480 P
(43) Date of publication of application: 20.09.2017
(73) Proprietor: The Regents of the University of California, Oakland, CA 94607-5200 (US)
(72) Inventor: ILIOPOULOS, Dimitrios, Los Angeles, California 90024 (US)
(74) Representative: FRKelly
(86) International application number: PCT/US2015/059956
(87) International publication number: WO 2016/077347

(56) References cited:
- US-A1- 2012 083 596
- US-A1- 2012 083 596
- US-A1- 2012 093 936
- US-A1- 2013 143 764
- US-A1- 2013 143 764
- ANTHONY MITCHELL: "Stock list of PNAs miRNA inhibitor_Nov 2012", DOCSFORD LIBRARY, DOCSFORD LIBRARY , 26 January 2014 (2014-01-26), pages 1-6, XP009505618, Retrieved from the Internet: URL:http://www.docsford.com/document/52032 9
- C POLYTARCHOU ET AL: "Mir-214 as a Therapeutic Target in Ulcerative Colitis (UC)-Associated Colon cancer", HELICOBACTER, vol. 152, no. 5, 1 May 2012 (2012-05-01), pages S-184, XP55440672, England
- Ahmed Farid E.: "Diagnostic MicroRNA Markers to Screen for Sporadic Human Colon Cancer in Stool: I. Proof of Principle", , 1 January 2013 (2013-01-01), pages 93-114, XP55477860, Retrieved from the Internet: URL:http://cgp.iiarjournals.org/content/10 /3/93.full.pdf [retrieved on 2018-05-23]
- POLYTARCHOU ET AL.: 'Mir-214 as a therapeutic target in ulcerative colitis (UC)-associated colon cancer' GASTROENTEROLOGY vol. 142, no. 5, 01 May 2012, page S-184, XP055440672
- UEDA ET AL.: 'DNA methylation of microRNA-124a is a potential risk marker of colitis-associated cancer in patients with ulcerative colitis' DIGESTIVE DISEASES AND SCIENCES vol. 59, no. 10, 10 May 2014, pages 2444 - 2451, XP035390262 DOI: 10.1007/S10620-014-3193-4
- POLYTARCHOU ET AL.: 'MicroRNA214 is associated with progression of ulcerative colitis, and inhibition reduces development of colitis and colitis-associated cancer in mice' GASTROENTEROLOGY vol. 149, no. 4, 01 October 2015, pages 981 - 992, XP055440678 DOI: 10.1053/J.GASTRO.2015.05.057
- Christos Polytarchou ET AL: "MicroRNA214 Is Associated With Progression of Ulcerative Colitis, and Inhibition Reduces Development of Colitis and Colitis-Associated Cancer in Mice", Gastroenterology, vol. 149, no. 4, 1 October 2015 (2015-10-01), pages 981-992, XP055440678, US ISSN: 0016-5085, DOI: 10.1053/j.gastro.2015.05.057

## Description

### TECHNICAL FIELD OF THE INVENTION

The present invention is defined in claim 1-11 and relates generally to detection, diagnosis, and monitoring of ulcerative colitis and colitis-associated colon cancer. The invention more specifically pertains to use of miR-214 as a marker for diagnosis, prognosis, and monitoring of ulcerative colitis (UC) and UC-related dysplasia, and as a treatment target for UC and UC-related dysplasia, such as by use of a chemical inhibitor of miR-214.

### BACKGROUND OF THE INVENTION

There is an increasing incidence of UC potentially due to the westernized way of life, diet and environmental alterations. UC patients undergo phases of remission and relapse, but there is lack of reliable biomarkers that correlate with disease activity. Patients with long standing and extensive disease are at increased risk of developing colorectal cancer. Medical treatments, including TNFα-modulating agents, induce remission but are often limited by side effects, lymphoma, lack of response, or development of resistance. Despite medical therapy, surgical intervention is frequently required and patient's quality of life is decreased. Genome-wide association study (GWAS) revealed susceptibility loci and pathways that influence the risk of disease, but solely insufficient to determine specific causative roles, suggesting that other genetic and/or epigenetic alterations contribute to UC pathogenesis. MicroRNAs, small noncoding RNAs acting as master regulators of gene expression, primarily through binding in 3'-UTR of target mRNAs, are essential regulators of signaling pathways, contributing to the pathogenesis of inflammatory diseases, including IBD. MicroRNA signaling pathways link chronic inflammation to malignant transformation in breast and liver, however their role in UC development remains largely unexplored.

Moreover, it can be difficult to distinguish between the similar symptoms of gastrointestinal disorders, such as inflammatory bowel disease (IBD) and irritable bowel syndrome (IBS). IBS is a group of symptoms that include chronic abdominal pain or discomfort and diarrhea, constipation, or alternating bouts of the two. People with IBS are not at higher risk for colon cancer, nor are they more likely to develop IBD or other gastrointestinal diseases.

There is a need to identify improved methods for the detection and treatment of ulcerative colitis (UC) or UC-related dysplasia. There is also a need to distinguish UC from Crohn's disease or irrritable bowel syndrome (IBS).

US2012083596 discloses pharmaceutical compositions comprising short single stranded oligonucleotides, of length of between 8 and 17 nucleobases which are complementary to human microRNAs. The short oligonucleotides are particularly effective at alleviating miRNA repression in vivo. It is found that the incorporation of high affinity nucleotide analogues into the oligonucleotides results in highly effective anti-microRNA molecules which appear to function via the formation of almost irreversible duplexes with the miRNA target, rather than RNA cleavage based mechanisms, such as mechanisms associated with RNaseH or RISC.

C Polytarchou et al discloses Mir-214 as a therapeutic target in ulcerative colitis (UC)-associated colon cancer (AGA Abstracts, Helicobacter, vol. 152, no. 5, pages S-184).

### SUMMARY OF THE INVENTION

The invention provides a method for detection or diagnosis of ulcerative colitis (UC) in a subject. The method comprises contacting a specimen obtained from the subject with reagents for assaying for miR-214. The method further comprises measuring the amount of miR-214 present in the specimen as compared to a control sample. Presence of ulcerative colitis is determined when a 2-fold elevated amount of miR-214 is present in the specimen compared to the control sample. The measuring may further comprise measuring the amount of PTEN, and/or PDLIM2 present in the specimen, and wherein a decreased amount of PTEN and/or PDLIM2 is present in the specimen compared to the control sample is indicative of UC. There is disclosed a method of detecting or monitoring inflammatory disease by assaying for levels of miR-214, phosphatase and tensin homolog (PTEN), and for PDZ and LIM domain protein 2 (PDLIM2). The presence or risk of inflammatory disease is determined when an elevated amount of miR-214 is present in the specimen compared to the control sample, or when a decreased amount of PTEN and/or PDLIM2 is present in the specimen compared to the control sample. The methods can be used to distinguish between ulcerative colitis and Crohn's disease.

In one embodiment, the specimen is intestinal biopsy tissue, such as, for example, colon tissue, or intestinal fluid. In a typical embodiment, the measuring comprises polymerase chain reaction (PCR) assay, such as real-time PCR. Alternatively, the measuring comprises in situ hybridization. In another embodiment, the measuring comprises an immunoassay (e.g., for PTEN or PDLIM2).

The method for monitoring the efficacy of treatment of ulcerative colitis in a subject typically comprises contacting a specimen obtained from the subject at a first time point with reagents for assaying for miR-214 (and, optionally, PTEN and/or PDLIM2); contacting a specimen obtained from the subject at a second time point with reagents for assaying for miR-214 ( and, optionally, PTEN and/or PDLIM2), wherein the subject has been treated for ulcerative colitis prior to the second time point. The method further comprises measuring the amount of miR-214 (and, optionally, PTEN and/or PDLIM2) present in the specimens obtained at the first and second time points; and determining whether an increased or decreased amount of miR-214 ( and, optionally, PTEN and/or PDLIM2) is present in the specimen obtained at the second time point compared to the specimen obtained at the first time point, which decreased amount of miR-214 (and, optionally, increased amount of PTEN and/or PDLIM2), is indicative of effective amelioration of the ulcerative colitis (UC). The above method may be modified to monitor the progression of ulcerative colitis (UC) in a subject, optionally performed in the absence of treatment, by comparing measurements obtained at the two time points. An increase in the amount of miR-214 (or decrease in the amount of PTEN and/or PDLIM2) at the second time point compared to the first time point is indicative of disease progression.

The specimen can be blood or other bodily fluid, such as peritoneal fluid, or a tissue specimen. Typically, the specimen is intestinal fluid or tissue. Examples of specimens include intestinal biopsy tissue, such as colon biopsy. The measuring typically comprises PCR, in situ hybridization, or immunoassay.

The invention additionally provides an antisense oligonucleotide, which is SEQ ID NO: 1 (TGTCTGTGCCTGCTG), that specifically binds to miR-214 (ACAGCAGGCA CAGACAGGCAGU; SEQ ID NO: 2);wherein the oligonucleotide comprises a locked nucleic acid and phosphorothioate linkages for use in a method of treating ulcerative colitis (UC) or UC-related dysplasia, or colitis-associated colon cancer, in a subject. The use comprises administering to the subject a therapeutically effective amount of an inhibitor of miR-214 that is an an antisense miR-214 oligonucleotide. The administering may be intracolonic or intravenous. The antisense oligonucleotide is provided in a more stabilized form by having locked nucleic acid and phosphorothioate linkages. The use can comprise administering the antisense miR-214 oligonucleotide either directly, or via a lentiviral vector.

### BRIEF DESCRIPTION OF THE DRAWINGS

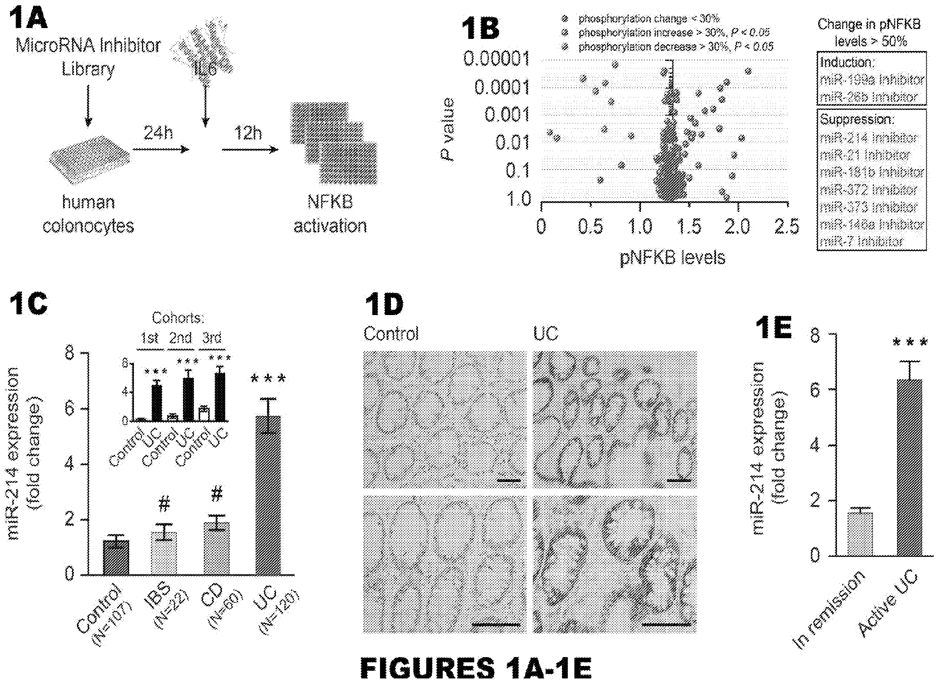

Figures 1A-1E show that high-content inhibitor screening identifies microRNAs that control NFκB in ulcerative colitis. Figs. 1A-1B, Screening workflow and data. Fig. 1A: A library of 348 microRNA inhibitors was transfected in colonic epithelial cells in triplicates and the activation of NFκB was measured after treatment with IL-6 and (Fig. 1B) screen data plotted as absolute levels (x- axis) of phosphorylated NFKB (on S536), compared to scrambled sequence controls (no effect, value=1.34) and P value from Student's t-test (y-axis). Box shows microRNA inhibitors that affect NFKB phosphorylation by more than 50%. Figs. 1C-1E, miR-214 is up-regulated in the colonic epithelium of human active ulcerative colitis samples. Fig. 1C: miR-214 is specifically overexpressed in colonic tissues from patients with UC, as demonstrated by quantitative polymerase chain reaction (qPCR). IBS, inflammatory bowel syndrome; CD, Crohn's disease; UC, ulcerative colitis. Data are represented as mean ± standard error of the mean (s.e.m.). ***P<0.001, in comparison to control; #P<0.001, in comparison to UC, Student's t-test. Fig. 1D: In situ hybridization, revealing miR-214 expression (blue) in the colonic epithelium UC patients and control. Nuclear Red was used as counterstain. Scale bars, 50 µm. Fig. 1E: miR-214 expression correlates with ulcerative colitis activity, as demonstrated by quantitative polymerase chain reaction (qPCR). Data are represented as mean ± standard error of the mean (s.e.m.). ***P<0.001, in colonic tissues from UC patients with active disease compared to UC patients in remission, Student's t-test.

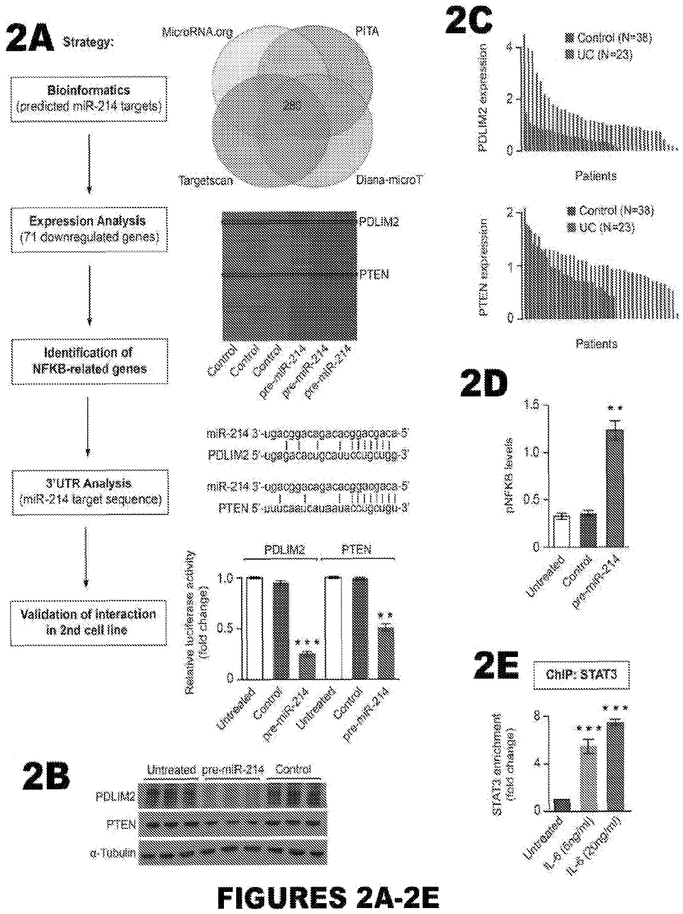

Figures 2A-2J show that miR-214 directly targets PDLIM2 and PTEN and activates an feedback loop circuit in the colonic epithelium. a, b, c, Strategy for the identification and validation of miR-214 targets regulating NFKB. (a) miR-214 targets predicted by 4 different prediction softwares (n=280) were analyzed by quantitative polymerase chain reaction (qPCR) in miR-214-treated human colonic epithelial cells (NCM356). The validated miR-214 targets (n=71, inhibited by more than 50% and ***P*<0.01, Student's *t*- test) were sifted based on relation to NFκB, the alignment of the 3'UTR of mRNAs (SEQ ID NO: 3, 4) and miR-214 (SEQ ID NO: 2) sequence, and confirmed by luciferase assays in a second colonic epithelial cell line (NCM460). (b) Western blot analysis revealed that miR-214 regulates the protein levels of PDLIM2 and PTEN in NCM356. (c) PDLIM2 (*P*<0.01) and PTEN (*P*<0.05) expression is decreased in colonic tissues from patients with UC, as demonstrated by quantitative polymerase chain reaction (qPCR). d, miR-214 regulates the phosphorylation of NFκB on S536, as demonstrated by ELISA. e, f, Inflammatory signals transcriptionally activate miR-214 through STAT3. IL6 induces the binding of STAT3 on the promoter of *MIR214* gene (e), while mutagenesis of the STAT3 binding site (M) abolishes promoter activation by IL6 (f). WT, wild type and M, mutated STAT3 binding site on *MIR214* gene promoter. Data are shown as the mean ± s.e.m. ** *P* < 0.01, *** *P* < 0.001 in comparison to untreated and control (pre-miR-scramble)-treated cells; ^{#} *P <* 0.001, in comparison to the IL6-induced WT promoter activity; Student's *t*-test. g, Schematic representation of the proposed model. h, i, j, A chemical inhibitor reverses the effects of miR-214 on colonic tissues from UC patients and ameliorates disease activity in an experimental mouse model of colitis. Fresh colonic biopsies isolated at colonoscopy, divided in two and treated with the chemical inhibitor against miR-214 or the negative control (NC) were subjected to analyses for miR-214 (h), PDLIM2 and PTEN mRNA levels (i). Effect of the chemical inhibitor against miR-214 on the disease activity in the chronic DSS mouse model of colitis. *** *P <* 0.001 in comparison to untreated and NC-treated tissues; ** *P <* 0.01 in comparison to untreated and NC-treated DSS mice; Student's *t*-test.

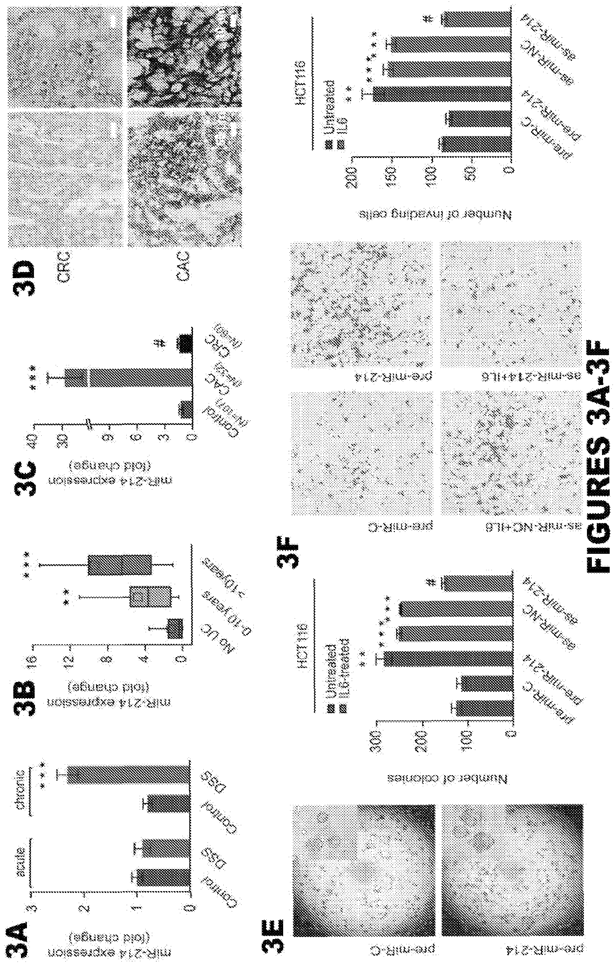

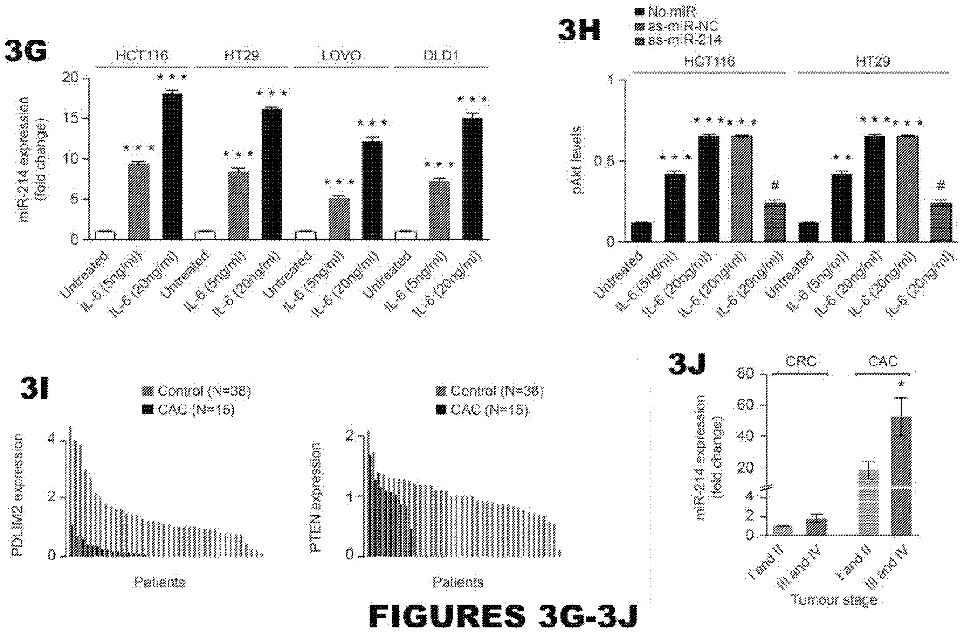

Figures 3A-3J illustrate the tumorigenic potential of miR-214 circuit in human colonocytes. Figs. 3A-3B: Chronic colonic inflammation regulates miR-214 expression in mice (Fig. 3A) and humans (Fig. 3B). Data are shown as the mean ± s.e.m. ** *P* < 0.01, *** *P* < 0.001 in comparison to control mice and control (No UC) tissues, Student's t test. Figs. 3C-3D: miR-214 is hyperexpressed in colitis associated colorectal cancer (CAC) but not sporadic colorectal cancer (CRC), as demonstrated by qPCR (Fig. 3C) and in situ hybridization (Fig. 3D). Data are shown as the mean ± s.e.m. *** *P <* 0.001, in comparison to control tissues; ^{#} *P <* 0.001, in comparison to CAC; Student's *t*-test. Figs. 3E-3H: IL-6 through miR-214 regulates the tumorigenic properties of colon cancer cells. miR-214 overexpression enhances, whereas miR-214 inhibition reverses the IL6-induced, colon cancer cell colony formation in soft agar (Fig. 3E) and invasion in matrigel (Fig. 3F). IL6 dose-dependently induces the expression of miR-214 (Fig. 3G), while miR-214 inhibition suppresses the effects of IL6 on the phosphorylation of Akt (Fig. 3H). Fig. 3I: PDLIM2 and PTEN mRNAs are suppressed in CAC and Fig. 3J, miR-214 positively correlates with tumour staging. Data are shown as the mean ± s.e.m. * *P* < 0.05, in comparison to early CAC stages (I and II); ** *P* < 0.01, *** *P <* 0.001 in comparison to untreated and control (miR-scramble)-treated cells; ^{#} *P* < 0.001 in comparison to anti-miR-negative control (NC)- treated cells; Student's t test.

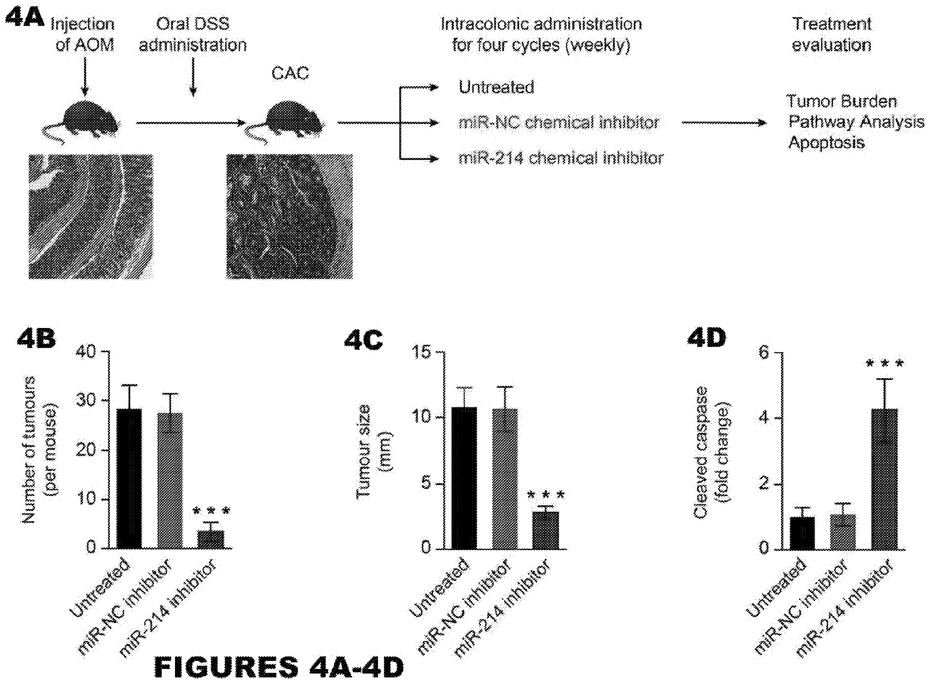

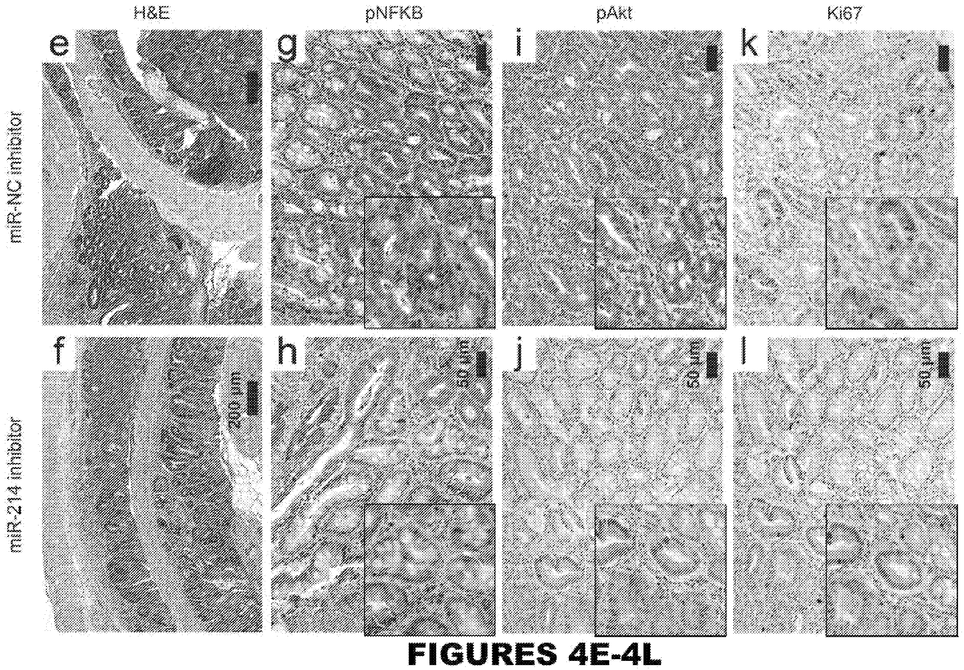

Figures 4A-4L show that a chemical inhibitor of miR-214 effectively suppresses colitis-associated colorectal cancer in vivo. Fig. 4A: Protocol for anti-miR-214 therapy in the mouse AOM-DSS colitis-associated colorectal cancer model. Figs. 4B-4D: Evaluation of the therapeutic potential of miR-214-inhibitor intracolonical administration on the (Fig. 4B) number and (Fig. 4C) size of tumours, and (Fig. 4D) the levels of cleaved caspase 3 in the tumours formed in the AOM-DSS mouse model of colitis-associated colorectal cancer. Data are shown as the mean ± s.e.m. *** *P* < 0.001 in comparison to untreated and control (miR-NC inhibitor)- administered mice, Student's *t*-test. Figs. 4E-4L: Haematoxylin/eosin (Figs. 4E-4F), pNFKB (S536) (Figs. 4G-4H), pAkt (S473) (Figs. 4I-4J), and Ki67 (Figs. 4K-4L) stained sections of colonic tissues from mice treated with the chemical miR-214 inhibitor or negative control (NC).

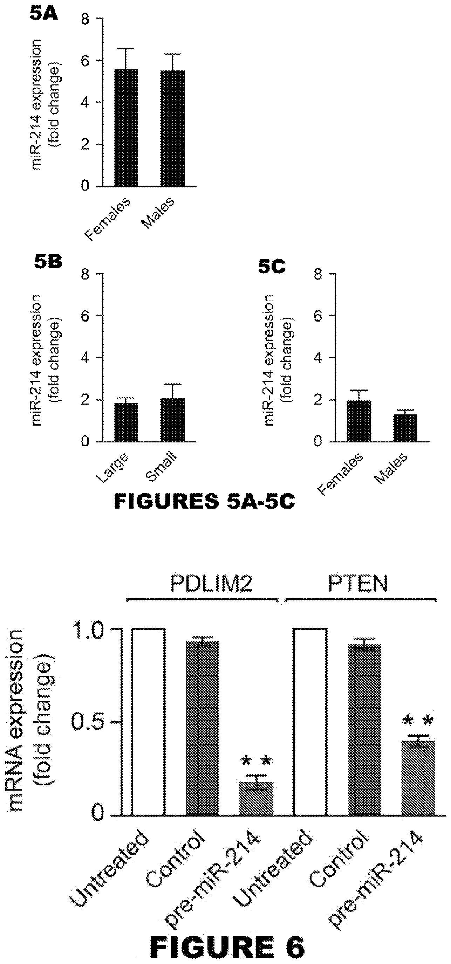

Figures 5A-5D are bar graphs showing miR-214 expression in patients with ulcerative colitis and Crohn's disease. MiR-214 expression, as assessed by qRT-PCR, (Fig. 5A) Expression of miR-214 in UC patients does not correlate to gender and does not differ in CD patients based on (Fig. 5B) disease location or (Fig. 5C) gender. Data are shown as the mean ± s.e.m.
Figure 6 is a bar graph showing that miR-214 regulates the expression of PDLIM2 and PTEN in colonic epithelial cells. Effect of miR-214 on the expression of PDLIM2 and PTEN in NCM460 cells, as assessed by qRT-PCR. Data are shown as the mean ± s.e.m. *** *P* < 0.001 in comparison to untreated and control (scramble) miR-treated cells, Student's t-test.

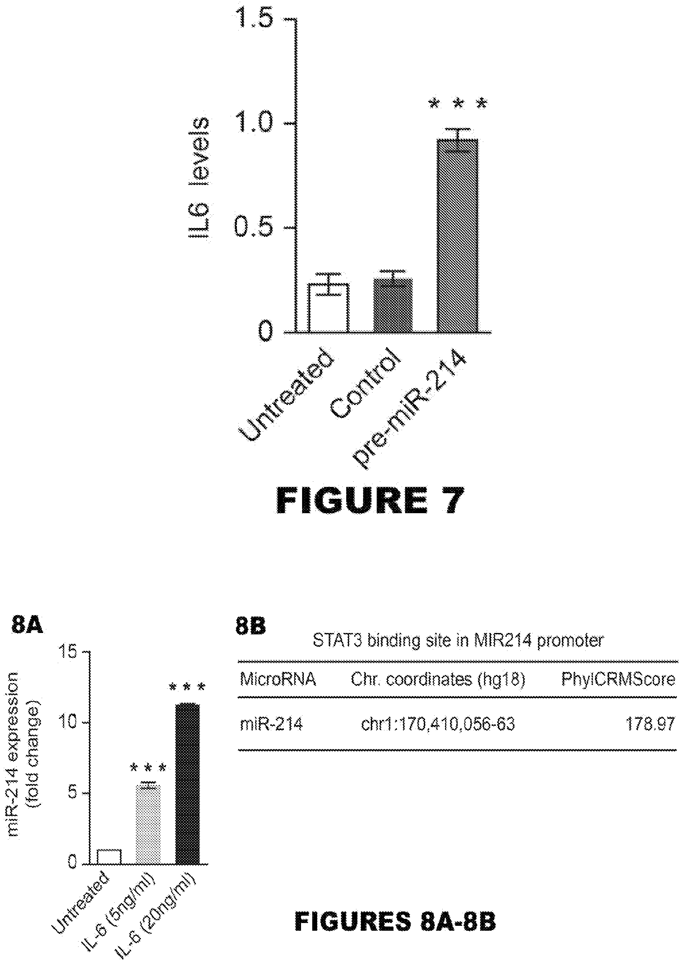

Figure 7 is a bar graph showing that miR-214 regulates the expression of IL6 in colonic epithelial cells. Effect of miR-214 on the expression of IL6 in NCM460 cells, as assessed by qRT-PCR. Data are shown as the mean ± s.e.m. *** P < 0.001 in comparison to untreated and control (scramble) miR-treated cells, Student's t-test.
Figures 8A-8B show that inflammatory signals transcriptionally activate miR-214 through STAT3. (Fig. 8A) Effects of IL6 on the expression of miR-214, as assessed by qRT-PCR. *** *P* < 0.001 in comparison to untreated and control (scramble) miR-treated cells, Student's t-test. Fig. 8B shows the outcome of Lever and PhylCRM algorithms that were employed for the prediction of STAT3 binding sites on the promoter of *MIR214* gene.

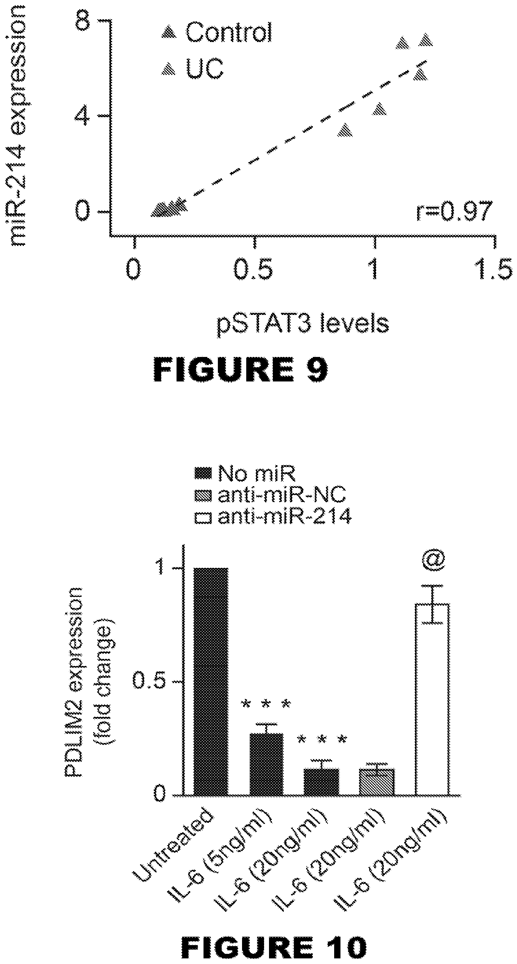

Figure 9 is a graph showing that the phosphorylation of STAT3 (on Y705) positively correlates to miR-214 expression in colonic tissues from control and patients with UC. STAT3 phosphorylation, as assessed by ELISA, and its correlation to miR-214 levels, as assessed by qRT-PCR, in colonic tissues. (r, Pearson's correlation).
Figure 10 is a bar graph showing that IL6 suppresses PDLIM2 expression in colonocytes through miR-214. Effect of miR-214 inhibition on IL6-induced PDLIM2 suppression, as assessed by qRT-PCR. *** *P* <0.001 in comparison to untreated cells, @ *P <* 0.001 in comparison to negative control miR-treated cells, Student's t-test.

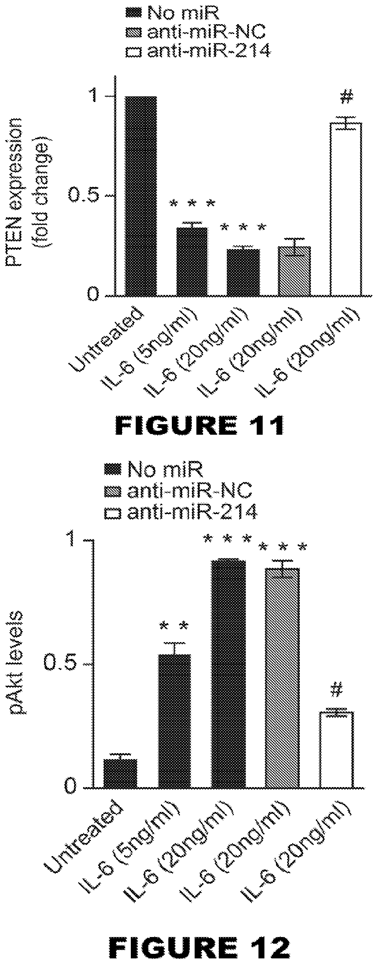

Figure 11 is a bar graph showing that IL6 suppresses PTEN expression in colonocytes through miR-214. Effect of miR-214 inhibition on IL6-induced PTEN suppression, as assessed by qRT-PCR. *** *P* <0.001 in comparison to untreated cells, # *P <* 0.01 in comparison to negative control miR-treated cells, Student's t-test.
Figure 12 is a bar graph showing that IL6 induces Akt phosphorylation in colonocytes through miR-214. Effect of miR-214 inhibition on IL6-induced Akt phosphorylation (S473), as assessed by ELISA. ** *P* <0.01, *** *P <* 0.001 in comparison to untreated cells, # *P <* 0.001 in comparison to negative control miR-treated cells, Student's t-test.

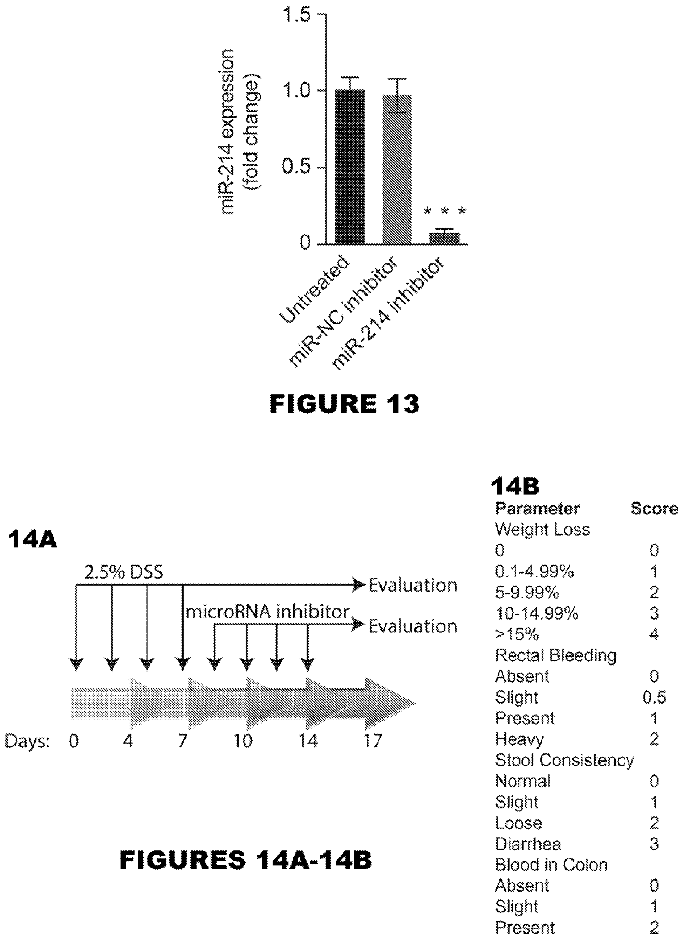

Figure 13 is a bar graph showing that a chemical inhibitor effectively suppresses miR-214 expression in colonocytes *in vitro.* The effectiveness of a miR-214-specific chemical inhibitor was tested *in vitro* in NCM356 colonocytes. Cells treated with the miR-214 chemical inhibitor (for 30 min) were analyzed for miR-214 levels, as assessed by qRT-PCR, 24 hours later.
Figures 14A-14B illustrate the therapeutic protocol applied on the chronic DSS mouse model. To address the therapeutic potential of miR-214 inhibition we used a mouse model of chronic colitis. In this model, colitis is induced by repeated oral administration of dextran sulfate sodium (DSS). (Fig. 14A) miR-214 inhibitor or the negative control were intracolonically administered in DSS-treated mice after day 8 for four cycles (every 2 days). (Fig. 14B) The criteria used for the assessment of the disease activity score for each group of mice (untreated, miR-214 inhibitor or miR-NC inhibitor). On day 20, the mice were sacrificed, and colon length was measured.

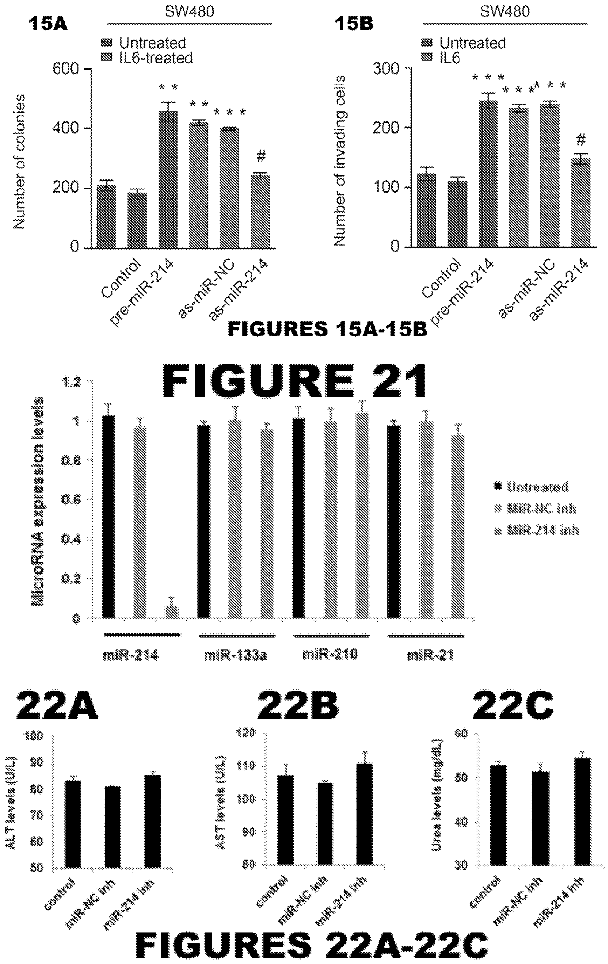

Figures 15A-15B are bar graphs showing that MiR-214 induces the tumorigenic potential of colon cancer cells and mediates the tumorigenic effects of IL6. MiR-214 overexpression enhances the tumorigenic potential, while miR-214 inhibition reverses the tumorigenic effects of IL6, on colon cancer cells. Effects of miR-214 overexpression and miR-214 inhibition on IL6-induced (Fig. 15A) colony formation in soft agar and (Fig. 15B) invasion of SW480 cells. Data are shown as the mean ± s.e.m. ** *P <* 0.01, *** *P <* 0.001 in comparison to untreated cells and control (miR-scramble)-treated cells and # *P <* 0.001 in comparison to anti-miR-negative control (NC)-treated cells, Student's t-test.

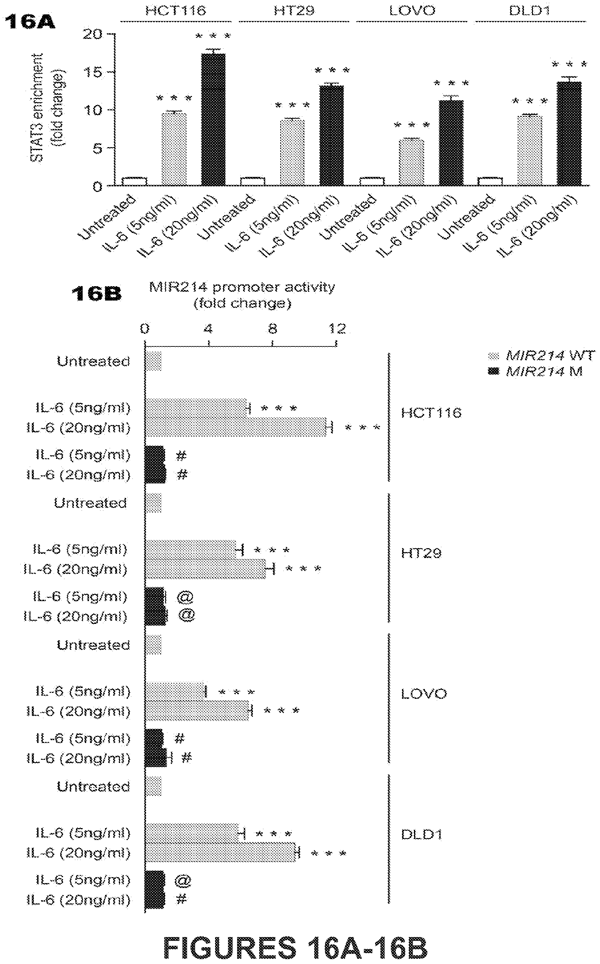

Figures 16A-16B are bar graphs showing that inflammatory signals transcriptionally activate miR-214 through STAT3 in colon cancer cells. (Fig. 16A) The binding of STAT3 on the promoter of *MIR214* gene in response to IL6 treatment, as assessed by ChIP and qPCR. (Fig. 16B) Luciferase activity of the *MIR214* gene promoter, bearing the wild-type (WT) or mutated (M) STAT3 binding site. Data are shown as the mean ± s.e.m. *** *P <* 0.001 in comparison to untreated cells. @ *P* < 0.01, # *P <* 0.001 in comparison to WT promoter activity under the respective treatment conditions, Student's t-test.

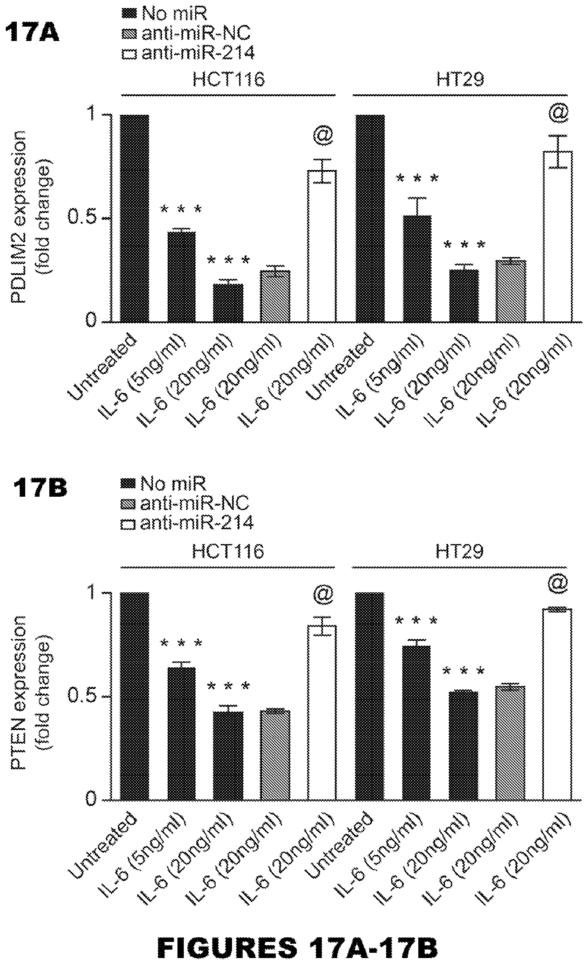

Figures 17A-17B are bar graphs showing that IL6 suppresses the expression of PDLIM2 and PTEN in colon cancer cells through miR-214. Effects of IL6 and inhibition of miR-214 on the expression of (Fig. 17A) PDLIM2 and (Fig. 17B) PTEN mRNAs, as assessed by qRT-PCR, in HCT116 and HT29 cells. Data are shown as the mean ± s.e.m. *** *P <* 0.001 in comparison to untreated cells. @ *P <* 0.01 in comparison to the anti-miR-negative control (NC)-treated cells, Student's *t*-test.

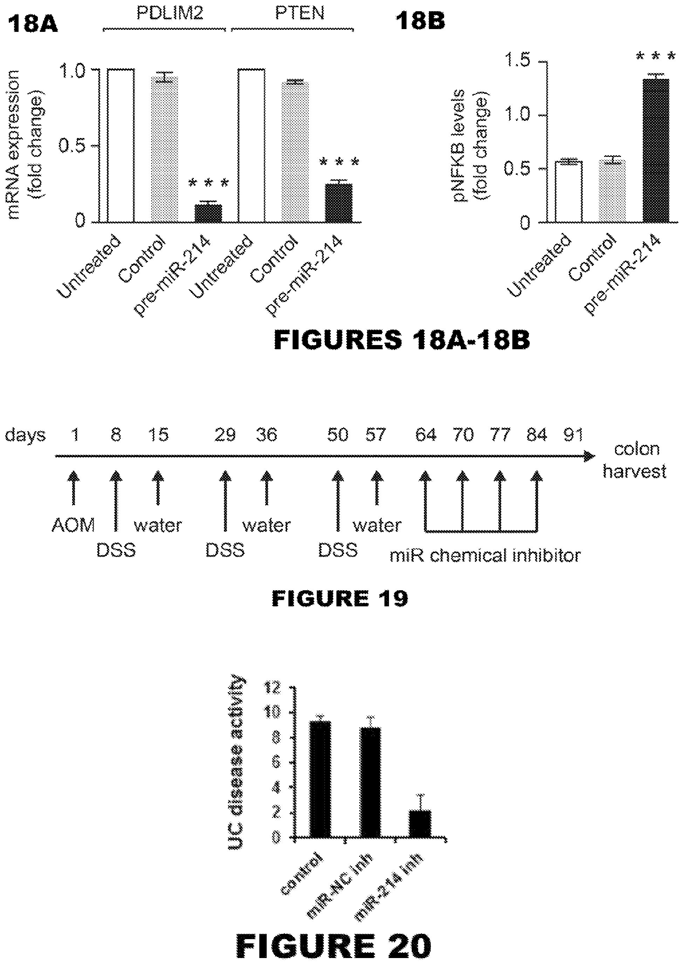

Figures 18A-18B are bar graphs showing that MiR-214 regulates the expression of PDLIM2 and PTEN and the phosphorylation of NFKB in colon cancer cells. Effects of miR-214 on the (Fig. 18A) expression of PDLIM2 and PTEN mRNAs, as assessed by qRT-PCR, and (Fig. 18B) phosphorylation of NFKB (on S536), as assessed by ELISA, in HCT116 cells. Data are shown as the mean ± s.e.m. *** *P <* 0.001 in comparison to untreated and control (scramble) miR-treated cells, Student's *t-*test.
Figure 19 illustrates the therapeutic protocol applied on AOM-DSS mouse model. To address the therapeutic potential of miR-214 inhibition we used a mouse model of colitis-associated colorectal cancer. In this model colorectal cancer is induced by administration of azoxymethane (AOM) followed by repeated oral administration of dextran sulfate sodium (DSS). Chemical microRNA negative control (miR-NC) or miR-214 inhibitor were intracolonically administered in AOM-DSS-treated mice on a weekly basis for four cycles. On day 91, the mice were sacrificed, and tumor burden was assessed.
Figure 20 is a bar graph showing that intracolonic administration of 18mg/kg of the miR-214 inhibitor, reduced 80% the ulcerative colitis disease activity in a mouse model of UC.
Figure 21 is a bar graph showing that the miR-214 inhibitor did not affect the expression levels of other, randomly selected, microRNAs (miR-133a, miR-210, miR-21), confirming its specificity for targeting only miR-214.
Figures 22A-22C are bar graphs showing the absence of toxicity in mice (3 mice/group) treated with 18mg/kg of miR-214. Levels of ALT and AST liver enzymes, together with urea levels, were measured 48h post treatment. The levels of ALT (Fig. 22A), AST (Figs. 22B) and urea (Fig. 22C) were not statistically significant different between the untreated (control), the miR-NC inhibitor and the miR-214 inhibitor treated mice.

### DETAILED DESCRIPTION OF THE INVENTION

The invention described herein is based on the unexpected and specific overexpression of miR-214 in ulcerative colitis (UC). The invention is further based on the demonstration that intracolonic administration of anti-sense miR-214 reduces UC with a high level of efficacy and specificity, and without toxicity. The data show that miR-214 is an important mediator in UC and a target for treatment. The invention is particularly advantageous in that it provides methods that can be used to distinguish between ulcerative colitis and Crohn's disease.

### Definitions

All scientific and technical terms used in this application have meanings commonly used in the art unless otherwise specified. As used in this application, the following words or phrases have the meanings specified.

As used herein, a "specimen" from a subject means a specimen obtained from the subject that contains blood or blood-derived cells, other bodily fluid, such as intestinal fluid, or biopsy tissue. Examples of biopsy tissue include intestinal tissue, such as colon biopsy tissue.

As used herein, a "control sample" means a specimen that represents a normal, healthy condition. The specimen may be blood, serum, or other fluid or tissue understood by those skilled in the art to serve as a suitable control. A sample of normal colon tissue or intestinal fluid obtained from a healthy patient is a typical example of a control sample.

As used herein, the term "subject" includes any human or non-human animal. The term "non-human animal" includes all vertebrates, e.g., mammals and non-mammals, such as non-human primates, horses, sheep, dogs, cows, pigs, chickens, and other veterinary subjects.

As used herein, "a" or "an" means at least one, unless clearly indicated otherwise.

Methods of detecting ulcerative colitis and treating ulcerative colitis and colitis-associated cancer

The invention provides a method for detection or diagnosis of ulcerative colitis (UC) in a subject. The method comprises assaying a specimen obtained from the subject for miR-214, an oligonucleotide having the sequence: ACAGCAGGCA CAGACAGGCAGU (SEQ ID NO: 2). In a further embodiment, the method comprises measuring the amount of PTEN, and/or PDLIM2 present in the specimen, and wherein a decreased amount of PTEN and/or PDLIM2 is present in the specimen compared to the control sample is indicative of UC. The method may comprise assaying the specimen for two, three, or more markers, including phosphatase and tensin homolog (PTEN), and PDZ and LIM domain protein 2 (PDLIM2). The assaying typically comprises contacting the specimen with reagents specific for miR-214, PTEN, and/or PDLIM2, and measuring the amount of miR-214, PTEN, and/or PDLIM2 present in the specimen. Representative reagents suitable for such assays are described in the Examples below. For example, quantitative real-time PCR can be used to determine expression levels. In the examples below, miR-214 expression levels were normalized to levels of U6 small nuclear RNA (203904; Exiqon). Those skilled in the art can appreciate suitable alternatives to be used to normalize expression levels.

The disease detected in this manner includes ulcerative colitis (UC). In particular, this method can be used to detect ulcerative colitis (UC), and to distinguish UC from other conditions, such as Crohn's disease or irritable bowel syndrome (IBS). Thus, the invention additionally provides a method of specifically detecting UC. This method is particularly useful for cases in which a diagnosis of UC or Crohn's disease has not been determined. The method may comprise determining the status (active or inactive/remission) of UC in a patient, whereby detecting elevated levels of miR-214 compared to a control sample is indicative of active disease. Levels of miR-214 that are reduced relative to a prior measurement for the same patient, or not significantly higher than a control sample, are indicative of disease regression or remission.

The amount of miR-214, PTEN, and/or PDLIM2 present in the specimen is then compared to that present in a control sample. An elevated amount of miR-214, or decreased amount of PTEN and/or PDLIM2, present in the specimen compared to the control sample is indicative of inflammatory disease. Typically, the amount of increase or decrease in the presence of the marker (miR-214, PTEN, and/or PDLIM2) in the specimen obtained from a subject who has inflammatory disease is a statistically significant difference compared to a normal control sample.

The amount of miR-214 in a specimen obtained from a subject is elevated 2-fold compared to a normal control. Optionally, the amount of PTEN, and/or PDLIM2 is decreased to about half that of a normal control. The difference may be an increase (in the case of miR-214) or decrease (in the case of PTEN, and/or PDLIM2) of 10%, 20%, 30%, 40%, 50%, 75%, or 100% relative to normal control.

The measuring or assay for miR-214 can involve isolating microRNA from the specimen and/or performing a polymerase chain reaction (PCR) assay, such as real-time PCR, or other suitable PCR assay known in the art. Alternatively, the assay can be an in situ hybridization assay. The assay for PTEN, and/or PDLIM2 can be PCR or an immunoassay, such as enzyme-linked immunosorbent assay (ELISA), immunoblotting, radioimmunoassay, or other immunoassays known in the art. Such assays can be performed on biopsied tissue samples.

Probes for detection of miR-214, PTEN, and/or PDLIM2 can be detectably labelled. The probe may be labeled with a radioisotope, an enzyme, a fluorescent substance, a luminescent substance or biotin. In another embodiment, the 5' end of an oligonucleotide probe is labeled with a reporter fluorescent dye and the 3' end of the probe is labeled with a quencher dye. In some embodiments, the probe is an antibody and the detectable label is an antibody that binds PTEN, and/or PDLIM2, or a secondary antibody that binds a primary antibody. Representative antibodies are described in the Examples below. Reagents and kits, including antibodies, probes, PCR primers and related materials are commercially available.

The specimen is typically intestinal fluid or intestinal tissue, such as biopsy tissue. Other specimens may be obtained in accordance with the judgment of the treating physician. Specimens can be obtained from subjects using conventional means.

Those skilled in the art will appreciate additional variations suitable for the method of detecting UC or UC-related dysplasia through detection of miR-214 in a specimen, as it provides a means of monitoring to assess disease activity and response to treatment. This method can also be used to monitor levels of miR-214 in a sample from a patient undergoing treatment. The suitability of a therapeutic regimen for initial or continued treatment can be determined by monitoring miR-214 levels using this method. The extent of miR-214 present in a given patient or specimen can provide a prognostic indicator to guide treatment strategy. Accordingly, one can use information about the level of miR-214 present in a subject to assist in selecting an appropriate treatment protocol. For example, mesalamine treatment of ulcerative colitis could be monitored by miR-214 as a surrogate biomarker to quantitatively measure the level of persisting disease activity. If disease activity persists above an acceptable level, the clinician would consider increasing the treatment dose, or changing to a different therapeutic agent.

The invention additionally provides an antisense oligonucleotide, which is SEQ ID NO: 1 (TGTCTGTGCCTGCTG), that specifically binds to miR-214 (ACAGCAGGCA CAGACAGGCAGU; SEQ ID NO: 2);wherein the oligonucleotide comprises a locked nucleic acid and phosphorothioate linkages for use in a method of treating ulcerative colitis (UC) or UC-related dysplasia, or colitis-associated colorectal cancer in a subject. The use comprises administering to the subject a therapeutically effective amount of an inhibitor of miR-214. A therapeutically effective amount is an amount sufficient to ameliorate disease symptoms, including, but not limited to, inflammation, and IL-8 expression. The administering may be intracolonic or intravenous. Alternatively, the administering may be intratumoral. The inhibitor of miR-214 is an antisense miR-214 oligonucleotide, that is an antisense oligonucleotide directed against SEQ ID NO: 2, and having SEQ ID NO: 1 (5'-TGTCTGTGCCTGCTG-3'). The antisense oligonucleotide is provided in a more stabilized form, so that the antisense miR-214 oligonucleotide comprises locked nucleic acid and phosphorothioate linkages. Such modifications both stabilize the molecule and protect it from endonuclease cleavage, which serve to increase the stability and efficacy of the inhibitor *in vivo.* The use can comprise administering the antisense miR-214 oligonucleotide directly, or via a lentiviral vector. Use of a suitable delivery vector, such as a lentiviral or adenoviral vector, for example, may be selected to enhance the efficacy of intravenous administration.

Treatment of UC or colon cancer in a subject comprises administering a therapeutically effective amount of an inhibitor of miR-214, that is an antisense miR-214 oligonucleotide, to the subject. A therapeutically effective amount is an amount sufficient to ameliorate symptoms of disease, such as tumor growth and/or size, and IL-8 expression. The administering can be intravenous, intracolonic, and/or intratumoral. The inhibitor of miR-214 may be administered intraoperatively at the time of biopsy and/or tumor resection. Representative doses of 12 and 18 mg/kg for treating UC in a mouse model are described in the Examples below, and shown to be specific, effective and non-toxic. Those skilled in the art can use this information to guide selection of an effective dose amount and schedule to other subjects.

Treatment of UC or cancer can be administered in a single dose or as a series of doses administered over time. Dosage and treatment regimens can be determined by the treating physician, taking into account disease severity, patient condition, and other factors.

### Kits

For use in the diagnostic applications described herein, kits are also disclosed herein. Such kits can comprise a carrier, package or container that is compartmentalized to receive one or more containers such as vials, tubes, and the like, each of the container(s) comprising one of the separate elements to be used in the method. The antibodies, probes, primers, and other reagents of the kit may be provided in any suitable form, including frozen, lyophilized, or in a pharmaceutically acceptable buffer such as TBS or PBS. The kit may also include other reagents required for utilization of the reagents in vitro or in vivo such as buffers (i.e., TBS, PBS), blocking agents (solutions including nonfat dry milk, normal sera, Tween-20 Detergent, BSA, or casein), and / or detection reagents (i.e., goat anti-mouse IgG biotin, streptavidin-HRP conjugates, allophycocyanin, B-phycoerythrin, R- phycoerythrin, peroxidase, fluors (i.e., DyLight, Cy3, Cy5, FITC, HiLyte Fluor 555, HiLyte Fluor 647), and / or staining kits (i.e., ABC Staining Kit, Pierce)). Nucleotide probes may be labeled for detection. The kits may also include other reagents and / or instructions for using antibodies and other reagents in commonly utilized assays described above such as, for example, flow cytometric analysis, ELISA, immunoblotting (i.e., western blot), in situ detection, immunocytochemistry, immunohistochemistry.

The kit may provide the reagent in purified form. In another embodiment, the reagents are immunoreagents that are provided in biotinylated form either alone or along with an avidin-conjugated detection reagent (i.e., antibody). Alternatively, the kit may include a fluorescently labeled immunoreagent which may be used to directly detect antigen. Buffers and the like required for using any of these systems are well-known in the art and may be prepared by the end-user or provided as a component of the kit. The kit may also include a solid support containing positive- and negative-control protein and / or tissue samples. For example, kits for performing spotting or western blot-type assays may include control cell or tissue lysates for use in SDS-PAGE or nylon or other membranes containing pre-fixed control samples with additional space for experimental samples.

The kit will typically comprise the container described above and one or more other containers comprising materials desirable from a commercial and user standpoint, including buffers, diluents, filters, needles, syringes, and package inserts with instructions for use. In addition, a label can be provided on the container to indicate that the composition is used for a specific application, and can also indicate directions for use, such as those described above. Directions and or other information can also be included on an insert, which is included with the kit.

### Representative Applications of the Invention

The identification of novel targets and compounds regulating NFKB activity can be achieved by use of the materials and methods described herein. Specifically, the invention identifies novel microRNA targets and inhibitors regulating NFKB activity in human colonocytes. The NFKB pathway is found to be activated in many different types of cancer and auto-immune and inflammatory diseases, and these inhibitors can be used to treat these diseases by suppressing NFKB phosphorylation.

MiR-214 is a diagnostic biomarker that is specific for ulcerative colitis patients. Several studies have identified IBD biomarkers, however most of these biomarkers are not highly specific for UC, but they are also biomarkers for Crohn's disease and other GI diseases. Most identified biomarkers are general inflammatory biomarkers. The findings described herein verify the specificity of miR-214 biomarker derived from analysis of 3 different cohorts of patients. Examination of miR-214 levels may help clinicians diagnose a patient with UC and choose the appropriate therapy. This is particularly useful in cases where the available clinical tests cannot distinguish UC from Crohn's and other gastrointestinal diseases.

MiR-214 is a prognostic biomarker of UC disease activity. MiR-214 levels are higher in UC patients with active disease relative to those in remission. Evaluation of miR-214 levels can be used to monitor the disease status even in the absence of symptoms and evaluate if there is need for therapeutic intervention. MiR-214 is highly specific to UC and correlates with UC disease activity, and may also be used for predicting risk for ulcerative colitis patients to develop colon cancer.

Evaluation of microRNA levels in human FFPE tissue by in situ hybridization. Although protein levels could be examined by immunohistochemistry (IHC) in formalin-fixed paraffin embedded (FFPE) sections from patient samples, it is preferable to develop a protocol for evaluating microRNA levels in the same tissues. The invention provides an optimized in situ hybridization protocol, improving its efficacy and specificity. The protocol can be found in the examples below.

MiR-214 levels provide a biomarker identifying UC patients who have higher risk to develop colon cancer. This is a significant advantage, since there is nothing available for UC patients. Even if the colon tissue seems normal during the colonoscopy procedure, evaluation of miR-214 levels may be used to identify early molecular oncogenic alterations to help the clinician decide the appropriate time for removing this area of the colon by surgery. Patients with >10 years with colitis have increased risk for colon cancer and the only solution is surgery. Mir-214 is the first biomarker that has 100% specificity for colon cancer patients with UC history and 0% specificity for sporadic colon cancer patients. MiR-214 is ~30-fold increased in colitis-associated colorectal cancer (CAC) patients, which is a difference that can be easily and specifically identified by PCR in a prognostic test.

Development of an inhibitor towards miR-214 (SEQ ID NO: 2). The invention identifies the appropriate sequence (SEQ ID NO: 1) in order to target efficiently miR-214 expression.

Efficacy of miR-214 inhibitor to suppress colitis ex vivo (fresh colonic explants from UC patients). This is the first demonstration that targeting microRNAs has therapeutic potential ex vivo.

Targeting miR-214 as a novel therapy for ulcerative colitis patients. Inhibition of miR-214 by enema reduces colitis in a mouse model of experimental colitis. The therapeutic protocol can be found in detail in Figure 14.

Strategy to identify microRNA downstream targets that are disease relevant. Through a combination of bioinformatics, expression and 3'UTR analysis, the invention identifies miR-214 targets.

Identification of a gene signature regulated by miR-214 (see Table 3). The invention identifies direct downstream targets of miR-214. These genes provide markers for evaluating the specificity and effectiveness of miR-214 inhibition.

PTEN and PDLIM2 levels provide biomarkers for UC patients (see fig. 2c). Identification of reduced PDLIM2 and PTEN mRNA levels in UC patients relative to healthy controls provides a method for detecting and monitoring UC.

MiR-214 is a novel oncogene in colon cancer. The examples below provide an extensive analysis validating the oncogenic properties of miR-214 overexpression.

MiR-214 provides a biomarker for colon cancer progression in patients with a history of colitis (Fig. 3j). MiR-214 has increased expression levels in colon cancer patients at advanced stages (stages III, IV) relative to those in early stages (I, II). The invention thus provides a method of staging colon cancer whereby detecting a 10-20-fold increase in miR-214 is indicative of stage I-II colon cancer, while detecting a 40-fold or greater increase in miR-214 is indicative of stage III-IV colon cancer.

A method is disclosed for administration of microRNAs by enema in colon cancer, as demonstrated by animal models (see fig. 4a). Delivery of nucleic acids, including microRNAs and siRNAs, by intravenous injections results in multiple side effects and there is need to have a carrier (e.g. nanoparticles) that increases the specificity and efficacy. For patients with GI diseases, including UC and colon cancer, intracolonic (enema) delivery of microRNAs is highly efficient. This approach minimizes side effects, as there is no need for a particle carrier of the microRNA that could induce different side effects (e.g. immune responses).

MiR-214 inhibition provides a therapeutic strategy for colon cancer patients with a history of ulcerative colitis. MiR-214 inhibition suppresses colon cancer growth in vivo. Description of the specific therapeutic protocol can be found in Fig. 19.

### EXAMPLES

The following examples are presented to illustrate the present invention and to assist one of ordinary skill in making and using the same. The examples are not intended in any way to otherwise limit the scope of the invention.

### Example 1: Therapeutically targeting miR-214 circuit in ulcerative colitis and colitis-associated cancer

Inflammatory Bowel Diseases (IBD), consist of ulcerative colitis (UC) and Crohn's Disease (CD), which are characterized by activation of inflammatory responses, and patients with longstanding disease are at higher risk of developing colorectal cancer. Thus, the identification of novel molecular targets with therapeutic potential for UC and UC-related dysplasia are of major importance. MicroRNAs are deregulated in ulcerative colitis, but their mechanistic role and therapeutic value remains speculative. Here, using a high throughput functional suppressor screen of the human microRNAome, we identified miR-214 as master regulator of nuclear factor kappa beta (NF-κB). MiR-214 is amplified specifically in colonic tissues of patients with active and longstanding UC, and hyper- expressed in colitis-associated colorectal cancer (CAC). Integration of bioinformatic and genome-wide profiling analyses revealed that miR-214 by suppressing PDZ and LIM domain 2 (PDLIM2) and phosphatase and tensin homolog (PTEN) genes is amplified through a feedback loop circuit. A chemical inhibitor perturbed this circuit in colonic biopsies from UC patients ex vivo and intracolonic administration therapeutically suppressed UC and CAC in mice. Taken together, miR-214 correlates with disease activity and progression to colorectal cancer while its inhibition provides a novel therapeutic option for both UC and CAC patients.

UC development is characterized by activation of inflammatory pathways, including NF- κB signaling. To identify microRNAs that regulate NF-κB activity in human colonocytes, we performed a microRNA functional screen by targeting the human microRNAome in IL6-treated NCM-460 cells. (Fig.1a). In the primary screen, two microRNA inhibitors (miR-26b and 199a) induced, while seven inhibitors (miR-7, 146a, 373, 372, 181b, 21 and 214) suppressed NF-κB phosphorylation by >50%, P<0.05 (Fig. 1b). The miR-214 inhibitor was identified as the most effective suppressor (>90%). Examination of miR-214 levels in UC (n=120) and control (n=107) colonic tissues from 3 different cohorts (Tables 1 and 2) revealed that miR-214 levels are significantly increased in UC, but not other G! diseases, irritable bowel syndrome (IBS) (n=22) and CD (n=60) (Fig. 1c). In situ hybridization in human colonic tissues identified epithelial cells as the origin of miR-214 overexpression (Fig. 1d). To evaluate the clinical relevance, we examined miR-214 expression in correlation with clinicopathological parameters. MiR-214 levels were significantly higher in UC patients with active disease relative to patients in remission (Fig. 1e), but did not differ in relation to UC patient's gender neither to gender/disease location in CD patients (Fig. 5). These results demonstrate that MIR214 is an epithelial expressed gene that regulates NF-κB activity and is deregulated specifically in UC in correlation with disease activity.

To establish the link between miR-214 and UC development, we aimed to identify downstream gene targets that could mediate NF-κB activation. Our strategy consisted of 5 steps (Fig. 2a). We employed 4 different microRNA target prediction software and identified 280 common miR-214 gene targets. Their expression was analyzed by qPCR in miR-214-treated colonocytes. The validated 71 (Table 3) genes (expression <50%, P<0.01) were examined for their correlation with the NF-κB signaling pathway and direct interaction with miR-214 employing 3'UTR luciferase assays in a second colonic epithelial cell line. This comprehensive analysis revealed PDLIM2, a nuclear ubiquitin E3 ligase inhibitor of NF-κB activity15, and PTEN, a suppressor of the AKT signaling pathway16 previously shown to intervene with NF-κB activation17,18, as the top miR-214 regulated genes. MiR-214 overexpression inhibited 3'UTR luciferase activities and resulted in suppression of PTEN and PDLIM2 mRNA (Fig. 6) and protein levels (Fig. 2B). In reflection of the human relevance of these findings, PTEN and PDLIM2 mRNA levels were decreased in UC colonic tissues relative to controls (Fig. 2C), thus inversely correlated with miR-214 expression. Interestingly, overexpression of miR-214 induced NF-κB (s536) phosphorylation (Fig. 2D) and subsequently the expression of IL6 (Fig. 7), indicating that PTEN and PDLIM2 are direct downstream effectors of miR-214 involved in the regulation of the NF-κB inflammatory response.

**Table 1. Cohorts of patients analyzed for miR-214 expression**

| **Cohorts** | **Control** | **UC** | **CD** | **IBS** | **CAC** | **CRC** |
|---|---|---|---|---|---|---|
| **UCLA** | 43 | 18 | 24 | 22 | 6 | 18 |
| **Leiden** | 52 | 63 | 13 | | 22 | 27 |
| **Origene** | 12 | 39 | 23 | | 4 | 15 |
| **Total Patients** | **107** | **120** | **60** | **22** | **32** | **60** |

**Table 2. Characteristics of patients analyzed for miR-214 expression**

| **Groups** | **Control** | **UC** | **CD** | **IBS** | **CAC** | **CRC** |
|---|---|---|---|---|---|---|
| **No of Patients** | 107 | 120 | 60 | 22 | 32 | 60 |
| **Age-yr Median** | 42 | 39.5 | 33 | 38.5 | 55.5 | 70 |
| **Range** | 16-80 | 20-72 | 17-74 | 18-55 | 25-82 | 31-91 |
| **Missing Data** | 9 | 10 | | | | |
| **P value** | | ns | ns | ns | *** | *** |
| **Sex-no (%)** | | | | | | |
| **Female** | 31(29) | 41(34) | 15(25) | 11(50) | 18(56) | 28(47) |
| **Male** | 57(53) | 44(37) | 29(48) | 11(50) | 14(44) | 32(53 |
| **Missing Data** | 19(18) | 35(29) | 16(27) | | | |
| **P value** | | ns | ns | ns | ns | ns (0.192) |
| **Disease Duration** | | 11(0.3-30) | | | | |
| **TNM stage-no (%)** | | | | | | |
| **I** | | | | | 6(19) | 13(22) |
| **II** | | | | | 16(50) | 19(32) |
| **III** | | | | | 7(22) | 18(30) |
| **IV** | | | | | 3(9) | 10(17) |
| **P value** | | | | | | ns(0.390) |

| | | | | | | |
|---|---|---|---|---|---|---|
| ***P<0.001 between groups (Kruskal Wallis test); #P<0.001 in comparison to CAC (Wilcoxon two sample test). | | | | | | |

**Table 3. Expression of miR-214 targets vs Control (miR-scramble) in colonic epithelial cells**

| **Symbol** | **ID** | **Name** | **Fold Change** | **P<** |
|---|---|---|---|---|
| ABR | 29 | active BCR-related | -2.16 | *** |
| ACER3 | 55331 | alkalineceramidase3 | -1.67 | *** |
| ADSS | 159 | adenylosuccinate synthase | -3.57 | *** |
| AP3B1 | 8546 | adaptor-related protein complex 3, beta 1 subunit | -1.53 | ** |
| ARPC5L | 81873 | actin related protein 2/3 complex, subunit 5-like | -2.07 | *** |
| BAZ2A | 11176 | bromodomain adjacent to zinc finger domain, 2A | -1.79 | *** |
| BRPF3 | 27154 | bromodomain and PHD finger containing, 3 | -1.58 | *** |
| CD151 | 977 | CD151 molecule (Raph blood group) | -2.62 | *** |
| CDC25B | 994 | cell division cycle 25 homolog B (S. pombe) | -1.90 | *** |
| CDIPT | 10423 | CDP-diacylglycerol--inositol 3-phosphatidyltransferase | -1.79 | *** |
| CEP85 | 64793 | centrosomal protein 85kDa | -1.57 | *** |
| CHMP4B | 128866 | charged multivesicular body protein 4B | -1.69 | *** |
| CPEB4 | 80315 | cytoplasmic polyadenylation element binding protein 4 | -1.92 | *** |
| CTDSP1 | 58190 | CTD (carboxy-terminal domain, RNA polymerase II, polypeptide A) small phosphatase 1 | -1.70 | *** |
| CTSS | 1520 | cathepsin S | -2.12 | *** |
| DNAJC5 | 80331 | DnaJ (Hsp40) homolog, subfamily C, member 5 | -1.59 | *** |
| F8 | 2157 | coagulation factor VIII, procoagulant component | -1.62 | *** |
| FAM134A | 79137 | family with sequence similarity 134, member A | -3.66 | *** |
| FAM189B | 10712 | family with sequence similarity 189, member B | -1.99 | *** |
| GALNT7 | 51809 | UDP-N-acetyl-alpha-D-galactosamine: polypeptide N-acetylgalactosaminyltransferase 7 | -2.08 | *** |
| GLI3 | 2737 | GLI family zinc finger 3 | -2.13 | *** |
| HMG20A | 10363 | high mobility group 20A | -1.75 | *** |
| IGSF3 | 3321 | immunoglobulin superfamily, member 3 | -1.64 | *** |
| INO80C | 125476 | INO80 complex subunit C | -2.40 | *** |
| INTS2 | 57508 | integrator complex subunit 2 | -1.61 | *** |
| IPO11 | 51194 | importin 11 | -2.06 | *** |
| ITCH | 83737 | itchy E3 ubiquitin protein ligase | -1.67 | *** |
| KLHDC3 | 116138 | kelch domain containing 3 | -1.56 | *** |
| KLK10 | 5655 | kallikrein-related peptidase 10 | -1.71 | *** |
| LAPTM4B | 55353 | lysosomal protein transmembrane 4 beta | -1.84 | *** |
| LARP1 | 23367 | La ribonucleoprotein domain family, member 1 | -2.12 | *** |
| LEPROTL1 | 23484 | leptin receptor overlapping transcript-like 1 | -2.24 | *** |
| MED19 | 219541 | mediator complex subunit 19 | -1.90 | *** |
| MTM1 | 4534 | myotubularin 1 | -1.56 | *** |
| NAA15 | 80155 | N(alpha)-acetyltransferase 15, NatA auxiliary subunit | -2.94 | *** |
| OTUB1 | 55611 | OTU domain, ubiquitin aldehyde binding 1 | -1.59 | *** |
| PAPD5 | 64282 | PAP associated domain containing 5 | -1.73 | *** |
| PDLIM2 | 64236 | PDZ and LIM domain 2 (mystique) | -3.37 | *** |
| PGGT1B | 5229 | PGGT1B | 2.25 | *** |
| PLA2G15 | 23659 | phospholipase A2, group XV | -1.66 | *** |
| PLK4 | 10733 | polo-like kinase 4 | -1.55 | *** |
| POLE3 | 54107 | polo-like kinase 4 | -1.55 | *** |
| PPP2CB | 5516 | protein phosphatase 2, catalytic subunit, beta isozyme | -1.68 | *** |
| PPP6C | 5537 | protein phosphatase 6, catalytic subunit | -1.72 | *** |
| PSMD10 | 5716 | proteasome (prosome, macropain) 26S subunit, non-ATPase, 10 | -3.19 | *** |
| PTEN | 5728 | phosphatase and tensin homolog | -2.47 | *** |
| RAB14 | 51552 | RAB14, member RAS oncogene family | -2.54 | *** |
| RASSF5 | 83593 | Ras association (RaIGDS/AF-6) domain family member 5 | -1.52 | *** |
| RBM22 | 55696 | RNA binding motif protein 22 | -2.46 | *** |
| RNF115 | 27246 | ring finger protein 115 | -1.71 | *** |
| RPIA | 22934 | ribose 5-phosphate isomerase A | -1.57 | *** |

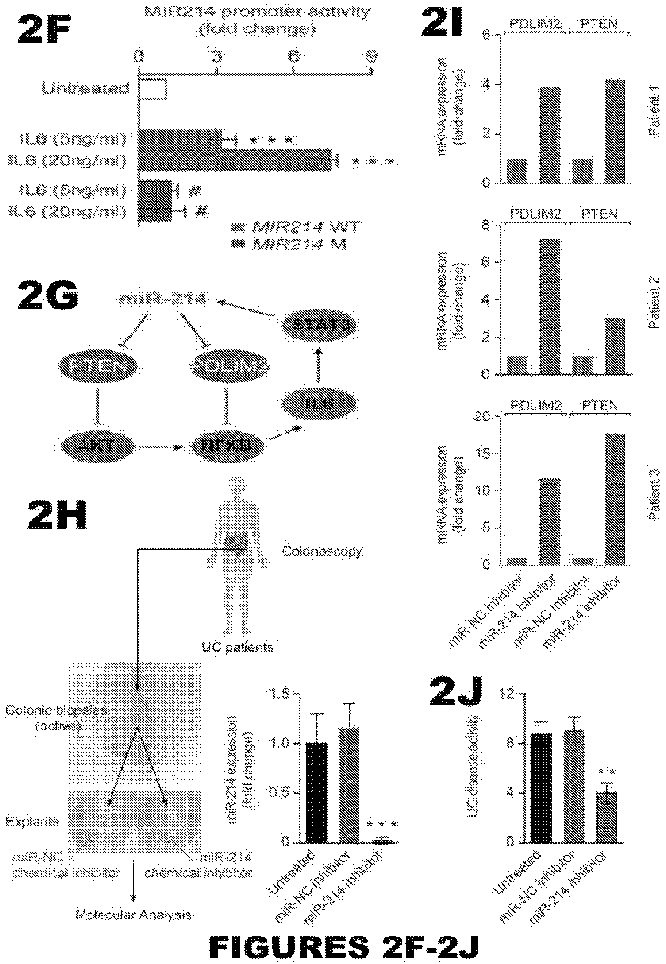

Given that miR-214 activates NF-κB and mediates IL6-induced NF-κB phosphorylation, we examined whether inflammatory signals regulate miR-214 expression. QPCR analysis revealed IL6 dose-dependent induction of miR-214 expression (Fig. 8A). Lever/PhylCRM algorithm analysis indicated the presence of STAT3 binding site in the promoter of MIR214 gene (Fig. 8B) and chromatin immunoprecipitation analyses revealed increased enrichment of STAT3 on MIR214 promoter upon treatment with IL6 (Fig. 2E). Similarly, IL6 dose-dependently induced MIR214 promoter activity, an effect abolished by mutation of the STAT3 binding site (Fig. 2F). The clinical importance of this interaction was evidenced by the correlation (r=0.97) of phospho-STAT3 to miR-214 levels (Fig. 9). The role of miR-214 as inflammatory mediator was studied in NCM356 colonocytes. IL6 dose- dependently suppressed PDLIM2 and PTEN expression an effect reversed by the inhibition of miR-214 (Figs. 10, 11). In accord, Akt phosphorylation was increased upon IL6 treatment, and reduced by miR-214 inhibition (Fig. 12). These findings suggest that IL6 induces miR-214 through STATS-mediated transcriptional activation, creating a positive feedback loop circuit (Fig. 2G).

To evaluate the therapeutic potential of miR-214 inhibition in UC through regulation of the feedback loop circuit, we used a chemically stable inhibitor, highly efficient in suppressing miR-214 in human colonocytes in vitro (Fig. 13). We studied the effects of the miR-214 chemical inhibitor ex vivo on freshly isolated colonic biopsies from UC patients with active disease. The inhibitor efficiently suppressed miR-214 in the colonic explants (Fig. 2H), and suppressed the inflammatory response by increasing the expression of both PDLIM2 and PTEN (Fig. 2I). Furthermore, miR-214 inhibitor reduced disease activity in an animal model of experimental colitis (Fig. 2J and Fig. 14). Taken together, these in vitro, in vivo and ex vivo data suggest the therapeutic potential of the miR-214 inhibitor in UC patients with active disease.

Although the association between UC and colorectal cancer has been documented as early as 1925 (19), its molecular biology remains to be explored. We questioned the role of miR-214 during the transition from UC to cancer. Interestingly, we found miR-214 is up-regulated in mice with chronic but not acute inflammation induced by DSS (Fig. 3A). In the same line, colonic tissues from patients with longstanding (>10 years) UC have increased miR-214 compared to those with <10 years (Fig. 3B), suggesting that miR-214 is a link between UC and colorectal cancer development. We compared miR-214 levels by qPCR and in situ hybridization in tumors from CAC and sporadic colon cancer (CRC) patients. This analysis showed the hyper-expression of miR-214 in CAC, but no difference between CRC and controls (Fig. 3C, 3D). Overall, these findings suggest that miR-214 is increased during UC development and amplified during progression to colorectal cancer.

To examine the importance of miR-214 in colorectal oncogenesis we performed gain- and loss-of function studies. Overexpression of miR-214 induced the colony formation ability and invasiveness of human colon cancer cells, suggesting its oncogenic role (Figs. 3E, 3F and Fig. 15). On the other hand, suppression of miR-214 inhibited the IL6-induced tumorigenic and invasive phenotype, pointing to the dependence of IL6 tumorigenic effects on miR-214. Next, we investigated whether miR-214 acts through regulation of the feedback loop circuit. IL6 dose-dependently up-regulated miR-214 in colon cancer cells (Fig. 3G), mediated by the binding of STAT3 in MIR214 promoter area (Fig. 16). IL6-induced miR-214 expression resulted in PDLIM2 and PTEN suppression (Fig. 17), Akt phosphorylation (Fig. 3H) and NF-κB activation (Fig. 18), suggesting that the miR-214 feedback loop circuit is hyper-activated during oncogenesis. In support of its human relevance, PDLIM2 and PTEN levels were decreased in tumors from CAC patients relative to controls (Fig. 3I). Given that miR-214 increases cancer cell aggressiveness, we examined miR-214 correlation to colon cancer progression. Examination of human tumors indicated that miR-214 is specifically up-regulated during CAC disease progression (Fig. 3J), providing additional evidence on the specificity of miR-214 circuit in UC and CAC. According to our data miR-214- positive feedback loop circuit links chronic inflammation to colon carcinogenesis. Therefore, targeting of miR-214 may be sufficient to suppress the development of CAC. We applied a therapeutic protocol consisting of intracolonic administration of a chemical miR-214 inhibitor or microRNA negative control for 4 cycles, weekly (Fig. 4A and Fig. 19) on the AOM- DSS mouse model. The miR-214 inhibitor reduced significantly the number (Fig. 4B) and size (Fig. 4C) of tumors. Mechanistically, miR-214 inhibitor suppressed tumor growth through the induction of apoptosis and activation of caspase-3 (Fig. 4D). In addition, immunohistochemical analysis revealed that miR-214 inhibition reduced NF-κB and Akt phosphorylation levels (Fig. 4E), indicating suppression of the miR-214-molecular circuit, and decreased the proliferation rate of colon cancer cells (Fig. 4E).

MiR-214 expression correlates with UC disease activity and disease duration and provides the molecular rationale for the current practice guidelines recommending surveillance colonoscopy 8 to 10 years after diagnosis of UC (20). Given that current surveillance procedures (21) and new imaging techniques (22-24) identify late-stage oncogenic events, miR-214 could serve as a biomarker, identifying UC patients at risk for malignant transformation. Furthermore, it is essential that although inflammatory pathways have been found to be activated in UC and CD, miR-214 is specific to UC. Intravenous administration of microRNA mimics has proven effective against liver cancer (8) and microRNA inhibitors are in Phase II-III clinical trials25. Here, we present a novel and efficient delivery method for microRNA therapeutics. Intracolonic administration of a chemically modified miR-214 inhibitor results in suppression of UC and CAC, suggesting the applicability of such approaches in patients with colitis and UC-related dysplasia. In conclusion, we demonstrate the UC-specific deregulation of miR-214 and its correlation with disease activity and duration, and identify a miR-214-driven inflammatory feedback loop circuit involved in the development of colonic oncogenesis. Tissue miR-214 could significantly alter current surveillance strategies and suppression of miR-214 could have therapeutic potential for colitis and colitis-associated colon cancer.

### Methods

The study was approved by the institutional review board at each study center. All patients from whom tissue samples were obtained at UCLA provided written informed consent. The patient samples were obtained at the Leiden University Medical Center according to the instructions and guidelines of the LUMC Medical Ethics Committee and in accordance with the Helsinki Declaration.

### MicroRNA library screen

NCM460 immortalized epithelial cells were plated in 96-well plates and transfected with a microRNA inhibitor library consisting of 348 microRNA inhibitors and 2 negative control microRNAs (100 nM) (Dharmacon Inc), as previously described¹². At 24 hours post-transfection, the cells were treated with IL6 for 24 hours, and the phosphorylation of NFKB was assessed by Phospho-ReIA/NFKB p65 (S536) Cell-Based ELISA (KCB7226, R&D). MicroRNA inhibitors that inhibited >50% the phosphorylation of NFKB were considered positive hits.

### Bioinformatic analysis

The Lever and PhylCRM algorithms have been used to identify STAT3 binding motifs in an area 5 kb upstream and 2 kb downstream of microRNAs.

### In situ hybridization

Double-DIG labeled miRCURY LNA Detection probe for the detection of miR-214 (38494-15, Exiqon) by *in situ* hybridization was used as previously described with modifications⁸. Sections of control, ulcerative colitis, and colorectal carcinomas were deparaffinized with xylene (three times for 5 min), followed by treatment with serial dilutions of ethanol (three times in 100%, twice in 96%, and three times in 70%) and by two changes of DEPC-PBS. Tissues were then digested with proteinase K (15 mg/ml) for 30 min at 37°C, rinsed three times with DEPC-PBS. Sections were dehydrated twice with 70%, 96%, and 100% ethanol, air-dried and hybridized for 1 hour with the hsa-miR-214 (40 nM) diluted in microRNA ISH buffer (90000, Exiqon), at 60°C. Following hybridization, sections were rinsed twice with 5XSSC, twice with 1XSSC, and three times with 0.2XSSC, 5 min each, at 60°C, and PBS. The slides were incubated with blocking solution (11585762001, Roche) for 15 min and then with anti-DIG antibody (1:800) in 2% sheep serum (013-000-121, Jackson Immunoresearch) blocking solution for 1 hour, at RT. Following three washes with PBS-T (PBS, 0.1% Tween-20), slides were incubated with the AP substrate buffer (NBT-BCIP tablet [11697471001, Roche] in 10 ml 0.2 mM Levamisole [31742, Fluka]) for 2 hours at 30°C in the dark. The reaction was stopped with two washes of AP stop solution (50 mM Tris- HCl, 150 mM NaCl, 10 mM KCI) and two washes with water. Tissues were counter-stained with Nuclear Fast Red for 1 min and rinsed with water. At the end, sections were dehydrated twice with 70%, 96%, and 100% ethanol and mounted with coverslips in Eukitt mounting medium (361894G, VWR). Images were captured with a Nikon 80i Upright Microscope equipped with a Nikon Digital Sight DS-Fi1 color camera, using the NIS-Elements image acquisition software. All images were captured and processed using identical settings.

### Immunohistochemistry

For tissue immunostaining for phospho-NFKB, phospho-Akt and Ki67, FFPE sections of colonic tissues from AOM-DSS mice treated with the chemical miR-214 inhibitor or negative control were deparaffinized with xylene (3x5 min) followed by treatment with serial dilutions of ethanol (100%, 100%, 95% and 95%, 10 min each) and by two changes of ddH₂O. Antigen unmasking was achieved by boiling the slides (95-99°C) for 10 min, in 10 mM sodium citrate, pH 6.0. Sections were rinsed three times with ddH2O, immersed in 3% H₂O₂ for 20 min, washed twice with ddH₂O and once with TBS-T (TBS, 0.1% Tween-20) and blocked for 1 hr with blocking solution (5% normal goat serum [5425, Cell Signaling Technology] in TBS-T). Phospho-P65 (Ser536) (SAB4300009, Sigma-Aldrich), phospho-Akt (Ser473) (4060, Cell Signaling Technology) and Ki67 (12202, Cell Signaling Technology) antibodies were diluted 1:200, 1:50 and 1:400, respectively, in Signal Stain antibody diluent (8112, Cell Signaling Technology) and incubated with the sections overnight at 4°C. Following incubation with the antibodies, sections were washed three times, 5 min each, with TBS-T and incubated for 1 hr at room temperature with SignalStain Boost ([HRP, Rabbit] 8114, or [HRP, Mouse] 8125, Cell Signaling Technology). Sections were washed three times, 5 min each, with TBS-T, and stained with the DAB Peroxidase Substrate Kit (SK-4100, Vector Laboratories) for 30 min, washed and counterstained with the hematoxylin QS (H-3404, Vector Laboratories). Finally, tissues were dehydrated and mounted in Eukitt medium. Microscope equipped with a Nikon Digital Sight DS-Fi1 color camera, using the NIS-Elements image acquisition software. All images were captured and processed using identical settings.

### Quantitative real-time PCR analysis

Real-time PCR was performed to determine the expression levels of miR-214 in human colon carcinomas and tissues. RNA was isolated, using Trizol (15596-026, Invitrogen). Reverse Transcription was carried out using the Universal cDNA synthesis kit (203300). Real-time PCR was carried out in triplicate using the SYBR Green master mix (203450) and primer for miR-214 (204510, Exiqon) in a CFX384 Real Time PCR detection system (BioRad). *MIR214* expression levels were normalized to the levels of U6 snRNA (203907, Exiqon).

Real-time PCR was employed to determine the expression levels of *PTEN and PDLIM2.* Reverse transcription was carried out using the Retroscript Kit (AM1710, Applied Biosystems). Real-time PCR for was carried out using IQ SYBR Green supermix (170-8882, BioRad). Glyceraldehyde-3- phosphate dehydrogenase (*GAPDH*) and *B-ACTIN* were used as the internal control. The sequences of the primers used are the following:
PTEN-F: 5'-cccagacatgacagccatc-3' (SEQ ID NO: 5)
PTEN-R: 5'-tctgcaggaaatcccatagc-3' (SEQ ID NO: 6)
PDLIM2-F: 5'-atggccacgattatgtctcc-3' (SEQ ID NO: 7)
PDLIM2-R: 5'-gcccatcatggtgactaagg-3' (SEQ ID NO: 8)
B-ACTIN-F: 5'-cccagcacaatgaagatcaa-3' (SEQ ID NO: 9)
B-ACTIN-R: 5'-acatctgctggaaggtggac-3' (SEQ ID NO: 10)
GAPDH-F: 5'-atgttcgtcatgggtgtgaa-3' (SEQ ID NO: 11)
*GAPDH-R:* 5'-ggtgctaagcagttggtggt-3' (SEQ ID NO: 12)

### Animal studies

Mouse studies were approved by the University of California Institutional Animal Care and Use *Committee* and conformed to the US National Institutes of Health Guide for the Care and Use of Laboratory Animals. The therapeutic potential of the miR-214 inhibition was tested in a mouse model of colitis-associated colon cancer (CAC). CAC is induced by administration of 12.5 mg/kg azoxymethane (AOM) at day 1 followed by repeated oral administration of 3% dextran sulfate sodium (DSS) at days 8, 29, 50. According to our treatment protocol, miR-NC inhibitor or miR-214 chemical inhibitor (12 mg/kg) were intracolonically administered in AOM-DSS-treated mice on a weekly basis for four cycles (days 64, 70, 77, 84). On day 91, the mice were sacrificed and the tumor burden was assessed. The miR-214 inhibitor that targeted the miR-214 sequence was 5'- TGTCTGTGCCTGCTG-3' (SEQ ID NO: 1).

### BioPlex ELISA assays

We used a sandwich ELISA assay to assess the phosphorylation status of Tyrosine 705 of STAT3 protein in control colonic tissues and samples from patients with ulcerative colitis and colitis- associated cancer, the levels of cleaved caspase-3 in colonic tumors derived from AOM-DSS- treated mice, the phosphorylation status of Serine 473 of Akt protein, the phosphorylation status of Serine 536 of NFKB protein and the secretion of IL6 in colonic cell lines. The data were analyzed in a BioPlex FlexMap3D (Bio-Rad) analyzer using the BioPlex Manager software.

### Luciferase assays

Colonic epithelial cells were transfected with a firefly luciferase reporter gene construct containing the promoter of *MIR214.* Cells were treated with IL6 24 hours after transfection of the luciferase vector. Cell extracts were prepared 24 later, and luciferase activity was measured using the Dual Luciferase Reporter Assay System (Promega, Wl, USA). For the 3'UTR assays, cells were transfected with the reporter vectors carrying the 3'UTR of *PTEN* or *PDLIM2.* At 24 hours they were transfected with miR-214 (or miR-scramble) and at 48 hours luciferase activity was measured using the Dual Luciferase Reporter Assay System.

### Invasion assays

We performed invasion assays in colonic epithelial cell lines 24 hours after transfection with miR-214 or anti-miR-214 or their respective controls. Invasion in matrigel has been conducted by using standardized conditions with BDBioCoat growth factor reduced MATRIGEL invasion chambers (PharMingen). Assays were conducted according to manufacturer's protocol, using 2% FBS as chemoattractant. Non-invading cells on the top side of the membrane were removed while invading cells were fixed and stained with 4'-6-diamidino-2-phenylindole (DAPI, Vector Laboratories Inc.), 16h post seeding. In all assays, 10 fields per insert were scored and se was calculated.

### Colony formation assays

Colonic epithelial cells were transfected with miR-214 or anti-miR-214 or their respective controls. Then, triplicate samples of 10⁵ cells from each cell line were mixed 4:1 (v/v) with 2.0% agarose in growth medium for a final concentration of 0.4% agarose. The cell mixture was plated on top of a solidified layer of 0.8% agarose in growth medium. The number of colonies was counted after 12 days.

### Chromatin immunoprecipitation

Chromatin immunoprecipitation was carried out as described previously⁸. Briefly, the chromatin fragments, derived from untreated and IL6-treated colonic epithelial cells, were immunoprecipitated with 6ug of antibody against STAT3. DNA extraction was performed using QIAGEN Purification Kit. Real-time PCR analysis was performed for STAT3 binding site in the miR-214 promoter using the following primers: forward: 5'-GGGCTTGAGTCCATCAGCTT-3' (SEQ ID NO: 13) and reverse 5'-GTTCAGCAGGACAGGTCTCA-3' (SEQ ID NO: 14) (Product size: 94 bp).

### Statistical analysis

All experiments were performed in triplicate unless otherwise stated. Statistical analyses were performed with the use of Origin software, version 8.6 and SAS statistical package version 9.4. Student's t-test was used to examine the statistical difference in miR-214 expression between control colonic tissues and specimens derived from different intestinal pathologies, between active and inactive UC specimens and between UC specimens categorized based on disease duration. The Kruskal Wallis test was used for age comparison and posthoc Wilcoxon two sample test with Bonferroni significance level adjustment for multiple testing were used for age comparisons, Chi-square test for gender comparisons and Fisher's Exact test for tumour stage comparison. The correlation significance was determined by means of Spearman and Pearson correlation analyses. The Student's t-test was used for comparisons between the differentially treated cells and mice groups. A P value of 0.05 or less was considered to indicate statistical significance.

### References

1. Eader, J.A., et al. Gut 48, 526-535 (2001).
2. Ekbom, A., et al. The New England journal of medicine 323, 1228-1233 (1990).
3. Soderlund, S., et al. Gastroenterology 136, 1561-1567; quiz 1818-1569 (2009).
4. Cho, W., et al. Journal of the Korean Surgical Society 83, 135-140 (2012).
5. Guo, H., et al. Nature 466, 835-840 (2010).
6. Boldin, M.P. & Baltimore, D. Immunological reviews 246, 205-220 (2012).
7. Androulidaki, A., et al. Immunity 31, 220-231 (2009).
8. Hatziapostolou, M., et al. Cell 147, 1233-1247 (2011).
9. Baltimore, D., et al. Nature immunology 9, 839-845 (2008).
10. Wu, F., et al. Gastroenterology 135, 1624-1635 e1624 (2008).
11. Stagakis, E., et al. Annals of the rheumatic diseases 70, 1496-1506 (2011).
12. Koukos, G., et al. Gastroenterology 145, 842-852 e842 (2013).
13. Iliopoulos, D., et al. Cell 139, 693-706 (2009).
14. Hatziapostolou, M., et al. Trends in endocrinology and metabolism: TEM 24, 361-373 (2013).
15. Tanaka, T., et al. Nature immunology 8, 584-591 (2007).
16. Polytarchou, C., et al. Cancer research 71, 4720-4731 (2011).
17. Romashkova, J.A. & Makarov, S.S. Nature 401, 86-90 (1999).
18. Dar, H.C., et al. Genes & development 22, 1490-1500 (2008).
19. Crohn, U.B. & Rosenberg, H. Am J Med Sci 170, 220-228 (1925).
20. Farraye, F.A., et al. Gastroenterology 138, 746-774, 774 e741-744; quiz e712-743 (2010).
21. Hurlstone, D.P., et al. Endoscopy 37, 1186-1192 (2005).
22. Kiesslich, R., et al. Gastroenterology 124, 880-888 (2003).
23. Dekker, E., et al. Endoscopy 39, 216-221 (2007).
24. Hurlstone, D.P., et al. Gut 57, 196-204 (2008).
25. Janssen, H.L., et al. The New England journal of medicine 368, 1685-1694 (2013).

### Example 2: Effectiveness of miR-214 inhibitor administered at 18mg/kg to suppress ulcerative colitis in mice

As described in Example 1 above, we evaluated the effectiveness of administering 12mg/kg of the miR-214 inhibitor intracolonically, to suppress ulcerative colitis in a chronic DSS mouse model (Figure 2i). Figures 14A-14B illustrates the therapeutic protocol applied on the chronic DSS mouse model. To address the therapeutic potential of miR-214 inhibition we used a mouse model of chronic colitis. In this model, colitis is induced by repeated oral administration of dextran sulfate sodium (DSS). (Fig. 14A) miR-214 inhibitor or the negative control were intracolonically administered in DSS-treated mice after day 8 for four cycles (every 2 days). (Fig. 14B) The criteria used for the assessment of the disease activity score for each group of mice (untreated, miR-214 inhibitor or miR-NC inhibitor). According to this experimental design, we found that 12mg/kg of the miR-214 inhibitor reduced by 50% the ulcerative colitis disease activity (Figure 2i).

We have used the same therapeutic protocol and increased the dose of the miR-214 inhibitor to 18mg/kg and evaluated its therapeutic potential in the chronic DSS mouse model. As shown below, we found that intracolonic administration of 18mg/kg of the miR-214 inhibitor, reduced 80% the ulcerative colitis disease activity, suggesting the effectiveness of this concentration to block colitis in mice. The results are shown in Figure 20.

### Example 3: Specificity of the miR-214 inhibitor administered at 18mg/kg in mice

To evaluate the specificity of the miR-inhibitor to block miR-214 expression levels, we evaluated miR-214 expression levels and found that the miR-214 inhibitor (administered intracolonically) blocked >95% miR-214 expression levels. To examine the specificity of these findings we examined the expression levels of other microRNAs (miR-133a, miR-210, miR-21). We found that the miR-214 inhibitor did not affect the expression levels of these randomly selected microRNAs, showing its specificity on targeting only miR-214. These results are shown in Figure 21.

### Example 4: Evaluation of toxicity effects of miR-214 inhibitor in mice

We examined if intracolonic administration of the miR-214 inhibitor has any potential toxic effects on liver and kidney function in mice. Specifically, mice (3 mice/group) were treated with 18mg/kg of miR-214 and levels of ALT and AST liver enzymes together with urea levels were measured 48h post treatment. As shown in the graphs of Figures 22A-22C, the levels of ALT, AST and urea were not statistically significant different between the untreated (control), the miR-NC inhibitor and the miR-214 inhibitor treated mice.

From the foregoing it will be appreciated that, although specific embodiments of the invention have been described herein for purposes of illustration, various modifications may be made without deviating from the scope of the invention. Accordingly, the invention is not limited except as by the appended claims.

## Claims

1. A method for detection or diagnosis of ulcerative colitis (UC) in a subject, the method comprising:
(a) measuring the amount of miR-214 present in a specimen obtained from the subject;
(b) comparing the amount of miR-214 present in the specimen to a control sample, wherein the control sample is a sample of normal colon tissue or intestinal fluid obtained from a healthy patient; and
(c) determining the presence or risk of UC when an elevated amount of miR-214 is present in the specimen compared to the control sample, wherein the elevated amount of miR-214 in the specimen obtained from the subject is elevated 2-fold compared to the control sample.

2. A method for monitoring the efficacy of treatment of UC in a subject, the method comprising:
(a) contacting a specimen obtained from the subject at a first time point with reagents for assaying for miR-214;
(b) contacting a specimen obtained from the subject at a second time point with reagents for assaying for miR-214, wherein the subject has been treated for UC prior to the second time point;
(c) measuring the amount of miR-214 present in the specimens obtained at the first and second time points; and
(d) determining whether a decreased amount of miR-214 is present in the specimen obtained at the second time point compared to the specimen obtained at the first time point, which decreased amount of miR-214 is indicative of effective amelioration of the UC.

3. The method of claim 1 or 2, wherein the measuring step further comprises measuring the amount of PTEN, and/or PDLIM2 present in the specimen, and wherein a decreased amount of PTEN and/or PDLIM2 is present in the specimen compared to the control sample is indicative of UC.

4. The method of any one of claims 1-3, wherein the measuring step comprises polymerase chain reaction assay.

5. The method of any one of claim 1 or 2, wherein the measuring step comprises detecting the binding of a probe that specifically binds to ACAGCAGGCACAGACAGGCAGU (SEQ ID NO: 2).

6. The method of claim 5, wherein the probe is an oligonucleotide which is SEQ ID NO: 1 (TGTCTGTGCCTGCTG).

7. The method of any one of claims 1 and 3-6, wherein the subject has been suffering from inflammatory bowel disease.

8. The method of any one of claims 1-7, wherein the specimen comprises intestinal biopsy tissue or intestinal fluid.

9. An antisense oligonucleotide which is SEQ ID NO: 1 (TGTCTGTGCCTGCTG) that specifically binds to miR-214 (ACAGCAGGCA CAGACAGGCAGU; SEQ ID NO: 2), wherein the oligonucleotide comprises a locked nucleic acid and phosphorothioate linkages, for use in a method of treating UC in a subject, the use comprising administering to the subject a therapeutically effective amount of the antisense oligonucleotide.

10. An antisense oligonucleotide which is SEQ ID NO: 1 (TGTCTGTGCCTGCTG) that specifically binds to miR-214 (ACAGCAGGCA CAGACAGGCAGU; SEQ ID NO: 2), wherein the oligonucleotide comprises a locked nucleic acid and phosphorothioate linkages, for use in a method of treating colitis associated colorectal cancer in a subject, the use comprising administering to the subject a therapeutically effective amount of the antisense oligonucleotide, wherein the administering is intracolonic.

11. The antisense oligonucleotide for use of claim 9 or 10, wherein the antisense oligonucleotide is administered via a lentiviral vector.

## Patentansprüche

1. Verfahren zur Erkennung oder Diagnose von Colitis ulcerosa (UC) bei einem Subjekt, wobei das Verfahren Folgendes umfasst:
(a) Messen der Menge an miR-214, die in einer von dem Subjekt erlangten Probe vorhanden ist;
(b) Vergleichen der in der Probe vorhandenen Menge an miR-214 mit einer Kontrollprobe, wobei die Kontrollprobe eine Probe von normalem Kolongewebe oder Darmfluid ist, die von einem gesunden Subjekt erlangt erhalten wurde; und
(c) Bestimmen des Vorliegens oder Risikos von UC, wenn in der Probe im Vergleich zu der Kontrollprobe eine erhöhte Menge an miR-214 vorhanden ist, wobei die erhöhte Menge an miR-214 in der von dem Subjekt erlangten Probe im Vergleich zu der Kontrollprobe um das Zweifache erhöht ist.

2. Verfahren zum Überwachen der Wirksamkeit der Behandlung von UC bei einem Subjekt, wobei das Verfahren Folgendes umfasst:
(a) Inkontaktbringen einer von dem Subjekt zu einem ersten Zeitpunkt erlangten Probe mit Reagenzien zum Testen auf miR-214;
(b) Inkontaktbringen einer von dem Subjekt zu einem zweiten Zeitpunkt erlangten Probe mit Reagenzien zum Testen auf miR-214, wobei das Subjekt vor dem zweiten Zeitpunkt wegen UC behandelt wurde;
(c) Messen der Menge an miR-214, die in den zu dem ersten und dem zweiten Zeitpunkt erhaltenen Proben vorhanden ist; und
(d) Bestimmen, ob in der zu dem zweiten Zeitpunkt erlangten Probe im Vergleich zu der zu dem ersten Zeitpunkt erlangten Probe eine verringerte Menge an miR-214 vorhanden ist, wobei eine verringerte Menge an miR-214 auf eine wirksame Linderung der UC hinweist.

3. Verfahren nach Anspruch 1 oder 2, wobei der Messschritt ferner Messen der in der Probe vorhandenen Menge an PTEN und/oder PDLIM2 umfasst und wobei das Vorhandensein einer verringerten Menge an PTEN und/oder PDLIM2 in der Probe im Vergleich zu der Kontrollprobe auf UC hinweist.

4. Verfahren nach einem der Ansprüche 1-3, wobei der Messschritt einen Polymerase-Kettenreaktionstest umfasst.

5. Verfahren nach einem der Ansprüche 1 oder 2, wobei der Messschritt Nachweisen der Bindung einer Sonde umfasst, die spezifisch an ACAGCAGGCACAGACAGGCAGU (SEQ ID NO: 2) bindet.

6. Verfahren nach Anspruch 5, wobei die Sonde ein Oligonukleotid der SEQ ID NO: 1 (TGTCTGTGCCTGCTG) ist.

7. Verfahren nach einem der Ansprüche 1 und 3-6, wobei das Subjekt an einer entzündlichen Darmerkrankung leidet.

8. Verfahren nach einem der Ansprüche 1-7, wobei die Probe Darmbiopsiegewebe oder Darmfluid umfasst.

9. Antisense-Oligonukleotid mit der SEQ ID NO: 1 (TGTCTGTGCCTGCTG), das spezifisch an miR-214 (ACAGCAGGCA CAGACAGGCAGU; SEQ ID NO: 2) bindet, wobei das Oligonukleotid eine LNA (locked nucleic acid) und Phosphorothioatbindungen umfasst, zur Verwendung in einem Verfahren zum Behandeln von UC bei einem Subjekt, wobei die Verwendung Verabreichen einer therapeutisch wirksamen Menge des Antisense-Oligonukleotids an das Subjekt umfasst.

10. Antisense-Oligonukleotid mit der SEQ ID NO: 1 (TGTCTGTGCCTGCTG), das spezifisch an miR-214 (ACAGCAGGCA CAGACAGGCAGU; SEQ ID NO: 2) bindet, wobei das Oligonukleotid eine LNA (locked nucleic acid) und Phosphorothioatbindungen umfasst, zur Verwendung in einem Verfahren zum Behandeln von Kolitis-assoziiertem kolorektalem Krebs bei einem Subjekt, wobei die Verwendung Verabreichen einer therapeutisch wirksamen Menge des Antisense-Oligonukleotids an das Subjekt umfasst, wobei das Verabreichen intrakolonisch erfolgt.

11. Antisense-Oligonukleotid zur Verwendung nach Anspruch 9 oder 10, wobei das Antisense-Oligonukleotid über einen lentiviralen Vektor verabreicht wird.

## Revendications

1. Procédé de détection ou de diagnostic de colite ulcéreuse (CU) chez un sujet, le procédé comprenant :
(a) la mesure de la quantité de miR-214 présente dans un prélèvement obtenu auprès du sujet ;
(b) la comparaison de la quantité de miR-214 présente dans le prélèvement à un échantillon témoin, dans lequel l'échantillon témoin est un échantillon de tissu de côlon normal ou de liquide intestinal obtenu auprès d'un patient en bonne santé ; et
(c) la détermination de la présence ou du risque de CU lorsqu'une quantité élevée de miR-214 est présente dans le prélèvement par rapport à l'échantillon témoin, dans lequel la quantité élevée de miR-214 dans le prélèvement obtenu auprès du sujet est multipliée par 2 par rapport à l'échantillon témoin.

2. Procédé de surveillance de l'efficacité d'un traitement de la CU chez un sujet, le procédé comprenant :
(a) la mise en contact d'un prélèvement obtenu auprès du sujet à un premier instant avec des réactifs pour le dosage de miR-214 ;
(b) la mise en contact d'un prélèvement obtenu auprès du sujet à un second instant avec des réactifs pour le dosage de miR-214, dans lequel le sujet a été traité pour la CU avant le second instant ;
(c) la mesure de la quantité de miR-214 présente dans les prélèvements obtenus aux premier et second instants ; et
(d) le fait de déterminer si une quantité réduite de miR-214 est présente dans le prélèvement obtenu au second instant par rapport au prélèvement obtenu au premier instant, laquelle quantité réduite de miR-214 est indicative d'une amélioration efficace de la CU.

3. Procédé selon la revendication 1 ou 2, dans lequel l'étape de mesure comprend en outre la mesure de la quantité de PTEN et/ou de PDLIM2 présente dans le prélèvement, et dans lequel une quantité réduite de PTEN et/ou de PDLIM2 présente dans le prélèvement par rapport à la quantité de PTEN et/ou de PDLIM2 présente dans l'échantillon témoin est indicative d'une CU.

4. Procédé selon l'une quelconque des revendications 1 à 3, dans lequel l'étape de mesure comprend un test de réaction en chaîne par polymérase.

5. Procédé selon l'une quelconque des revendications 1 ou 2, dans lequel l'étape de mesure comprend la détection de la liaison d'une sonde qui se lie spécifiquement à ACAGCAGGCACAGACAGGCAGU (SEQ ID NO : 2).

6. Procédé selon la revendication 5, dans lequel la sonde est un oligonucléotide qui est SEQ ID NO : 1 (TGTCTGTGCCTGCTG).

7. Procédé selon l'une quelconque des revendications 1 et 3 à 6, dans lequel le sujet souffre d'une maladie inflammatoire de l'intestin.

8. Procédé selon l'une quelconque des revendications 1 à 7, dans lequel le prélèvement comprend du tissu de biopsie intestinale ou du liquide intestinal.

9. Oligonucléotide antisens qui est SEQ ID NO : 1 (TGTCTGTGCCTGCTG) qui se lie spécifiquement à miR-214 (ACAGCAGGCA CAGACAGGCAGU ; SEQ ID NO : 2), dans lequel l'oligonucléotide comprend un acide nucléique verrouillé et des liaisons phosphorothioate, destiné à être utilisé dans un procédé de traitement de la CU chez un sujet, l'utilisation comprenant l'administration au sujet d'une quantité thérapeutiquement efficace de l'oligonucléotide antisens.

10. Oligonucléotide antisens qui est SEQ ID NO : 1 (TGTCTGTGCCTGCTG) qui se lie spécifiquement à miR-214 (ACAGCAGGCA CAGACAGGCAGU ; SEQ ID NO : 2), dans lequel l'oligonucléotide comprend un acide nucléique verrouillé et des liaisons phosphorothioate, destiné à être utilisé dans un procédé de traitement du cancer colorectal associé à la colite chez un sujet, l'utilisation comprenant l'administration au sujet d'une quantité thérapeutiquement efficace de l'oligonucléotide antisens, dans lequel l'administration se fait par voie intracolique.

11. Oligonucléotide antisens destiné à être utilisé selon la revendication 9 ou 10, dans lequel l'oligonucléotide antisens est administré par l'intermédiaire d'un vecteur lentiviral.
